# EUROPEAN PATENT SPECIFICATION

(11) **EP 1 792 983 B1**
(45) Date of publication and mention of the grant of the patent: **29.04.2009**
(21) Application number: 06011595.3
(22) Date of filing: 09.11.2000
(51) Int. Cl.: C12N 9/24, A61K 38/47, A61P 3/00

(54) **RECOMBINANT ALPHA-L-IDURONIDASE AND METHODS FOR TREATING DISEASES CAUSED BY DEFICIENCIES THEREOF**
REKOMBINANTE ALPHA-L-IDURONIDASE UND BEHANDLUNG VON DURCH DIESE HERVORGERUFENE MANGELERKRANKUNGEN
ALPHA-L-IDURONIDASE RECOMBINANTE ET METHODES DE TRAITEMENT DES MALADIES CAUSEES PAR DES DEFICIENCES DE CELLE-CI

(30) Priority: 12.11.1999 US 439923
(43) Date of publication of application: 06.06.2007
(62) Divisional of application: 00993857.2
(73) Proprietor: BioMarin Pharmaceutical Inc., Novato CA 94949 (US); Los Angeles Biomedical Research Institute at Harbor-UCLA Medical Center, Torrance, CA 90502 (US)
(72) Inventor: Henstrand, John M., Oakland, CA 94602 (US); Qin, Minmin, Pleasanton, CA 94588 (US); Chan, Wia-Pan, Castro Valley, CA 94546 (US); Chen, Lin, San Francisco, CA 94134 (US); Fitzpatrick, Paul A., Albany, CA 94706 (US); Wendt, Dan J., Walnut Creek, CA 94588 (US); Zecherle, Gary N., Novato, CA 94947 (US); Starr, Christopher M., Sonoma, CA 95476-0226 (US); Kakkis, Emil D., Novato, CA 94949 (US); Tanamachi, Becky, Signal Hill, CA 90807 (US)
(74) Representative: Harding, Charles Thomas

(56) References cited:
- WO-A-99/51724
- WO-A-99/58691
- KAKKIS E D ET AL: "Long-term and high-dose trial of enzyme replacement therapy in the canine model of mucopolysaccharidosis I" BIOCHEMICAL AND MOLECULAR MEDICINE, ORLANDO, FL, US, vol. 58, no. 2, August 1996 (1996-08), pages 156-167, XP000862844
- KAKKIS E D ET AL: "OVEREXPRESSION OF THE HUMAN LYSOSOMAL ENZYME ALPHA-L-IDURONIDASE IN CHINESE HAMSTER OVARY CELLS" PROTEIN EXPRESSION AND PURIFICATION, ACADEMIC PRESS, US, vol. 5, no. 3, June 1994 (1994-06), pages 225-232, XP000857380 ISSN: 1046-5928
- CLEMENTS P R ET AL: "Human alpha-L-iduronidase 1. Purification, monoclonal antibody production, native and subunit molecular mass" EUROPEAN JOURNAL OF BIOCHEMISTRY, vol. 152, no. 1, October 1985 (1985-10), pages 21-28, XP000857400
- UNGER E G ET AL: "Recombinant.alpha.-L-iduronidase: characterization of the purified enzyme and correction of mucopolysaccharidosis type I fibroblasts" BIOCHEMICAL JOURNAL, vol. 304, no. 1, November 1994 (1994-11), pages 43-49, XP000857388
- KAKKIS E D ET AL: "Enzyme-replacement therapy in mucopolysaccharidosis I." NEW ENGLAND JOURNAL OF MEDICINE, vol. 344, no. 3, 18 January 2001 (2001-01-18), pages 182-188, XP001030869

## Description

### FIELD OF THE INVENTION

The present invention is in the field of molecular biology, enzymology, biochemistry and clinical medicine. In particular, the present invention provides a human recombinant α-L-iduronidase, methods of large-scale production and purification of commercial grade human recombinant α-L-iduronidase enzyme, and methods to treat certain genetic disorders including α-L-iduronidase deficiency and mucopolysaccharidosis I (MPS I).

### BACKGROUND OF THE INVENTION

Carbohydrates play a number of important roles in the functioning of living organisms. In addition to their metabolic roles, carbohydrates are structural components of the human body covalently attached to numerous other entities such as proteins and lipids (called glycoconjugates). For example, human connective tissues and cell membranes comprise proteins, carbohydrates and a proteoglycan matrix. The carbohydrate portion of this proteoglycan matrix provides important properties to the body's stricture.

A genetic deficiency of the carbohydrate-cleaving, lysosomal enzyme α-L-iduronidase causes a lysosomal storage disorder known as mucopolysaccharidosis I (MPS I) (Neufeld and Muenzer, pp. 1565-1587, in The Metabolic Basis of Inherited Disease, Eds., C.R. Scriver, A.L. Beaudet, W.S. Sly, and D.Valle, McGraw-Hill, New York (1989)) In a severe form, MPS I is commonly known as Hurler syndrome and is associated with multiple problems such as mental retardation, clouding of the cornea, coarsened facial feature, cardiac disease, respiratory disease, liver and spleen enlargement, hernias, and joint stiffness. Patients suffering from Hurler syndrome usually die before age 10. In an intermediate form known as Hurler-Scheie syndrome, mental function is generally not severely affected, but physical problems may lead to death by the teens or twenties. Scheie syndrome is the mildest form of MPS I. It is compatible with a normal life span, but joint stiffness, corneal clouding and heart valve disease cause significant problems.

The frequency ofMPS I is estimated to be 1:100,000 according to a British Columbia survey of all newborns (Lowry, et al., Human Genetics 85:89-390 (1990)) and 1:70,000 according to an Irish study (Nelson, Human Genetics 101:355-358 (1990)). There appears to be no ethnic predilection for this disease. It is likely that worldwide the disease is underdiagnosed either because the patient dies of a complication before the diagnosis is made or because the milder forms of the syndrome may be mistaken for arthritis or missed entirely. Effective newborn screening for MPS I would likely find some previously undetected patients.

Except for a few patients which qualify for bone marrow transplantation, there are no significant therapies available for all MPS I patients. Hobbs, et al. (Lancet 2: 709-712 (1981)) first reported that bone marrow transplantation successfully treated a Hurler patient. Since that time, clinical studies at several transplant centers have shown improvement in physical disease and slowing or stabilizing of developmental decline if performed early. (Whitley, et al., Am. J. Med. Genet. 46: 209-218 (1993); Vellodi, et al., Arch. Dis. Child. 76: 92-99 (1997); Peters, et al., Blood 91: 2601-2608 (1998); Guffon, et al., J. Pediatrics 133: 119-125 (1998)) However, the significant morbidity and mortality, and the need for matched donor marrow, limits the utility of bone marrow transplants. An alternative therapy available to all affected patients would provide an important breakthrough in treating and managing this disease.

Enzyme replacement therapy has been considered a potential therapy for MPS I following the discovery that α-L-iduronidase can correct the enzymatic defect in Hurler cells in culture, but the development of human therapy has been technically unfeasible until now. In the corrective process, the enzyme containing a mannose-6-phosphate residue is taken up into cells through receptor-mediated endocytosis and transported to the lysosomes where it clears the stored substrates, heparan sulfate and dermatan sulfate. Application of this therapy to humans has previously not been possible due to inadequate sources of α-L-iduronidase in tissues.

For α-L-iduronidase enzyme therapy in MPS I, a recombinant source of enzyme has been needed in order to obtain therapeutically sufficient supplies of the enzyme. The cDNA for the canine enzyme was cloned in 1991 (Stoltzfus, et al., J. Biol. Chem. 267:6570-6575 (1992) and for the human enzyme in the same year. (Scott, et al., Proc. Natl. Acad. Sci. U.S.A. 88:9695-9699 (1991), Moskowitz, et al., FASEB J 6:77 (1992)). Following the cloning of cDNA for α-L-iduronidase, the production of adequate quantities of recombinant α-L-iduronidase allowed the study of enzyme replacement therapy in canine MPS L (Kakkis, et al., Protein Expr. Purif. 5: 225-232 (1994)) Enzyme replacement studies in the canine MPS I model demonstrated that intravenously-administered recombinant α-Liduronidase distributed widely and reduced lysosomal storage from many tissues. (Shull, et al., Proc. Natl. Acad. Sci. U.S.A. 91: 12937-12941 (1994); Kakkis, et al., Biochem. Mol. Med. 58: 156-167 (1996))

### BRIEF SUMMARY OF THE INVENTION

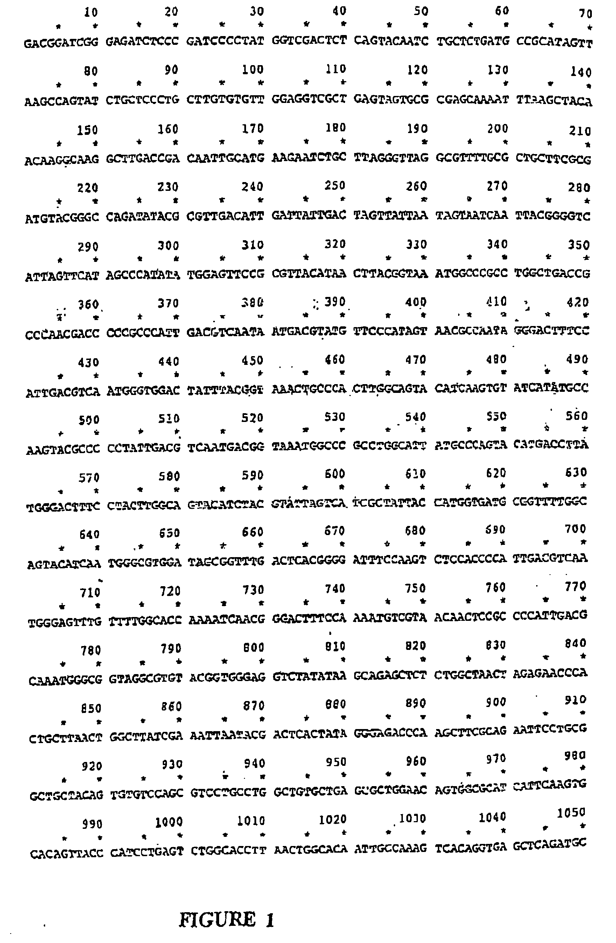

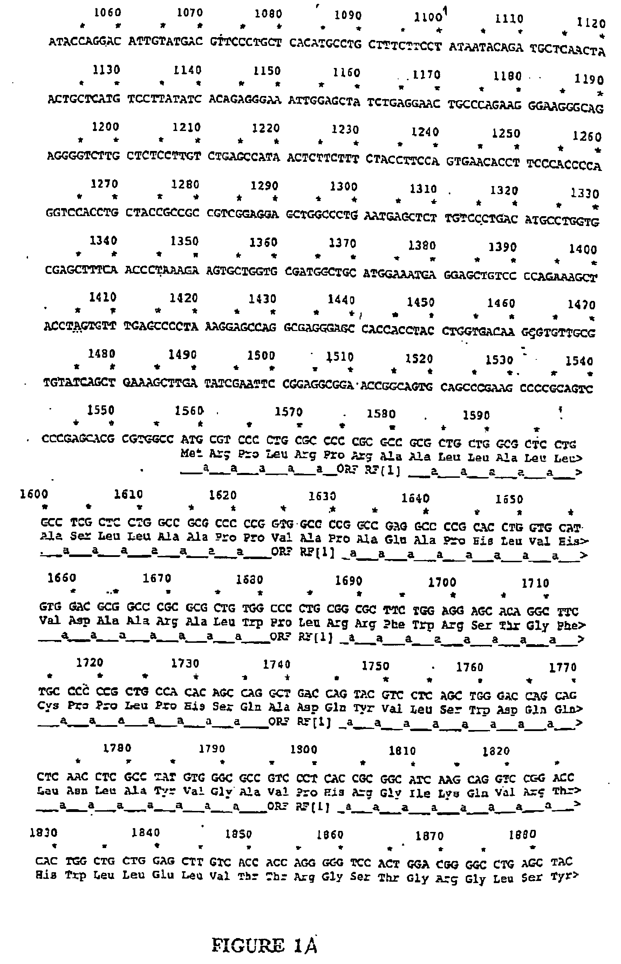

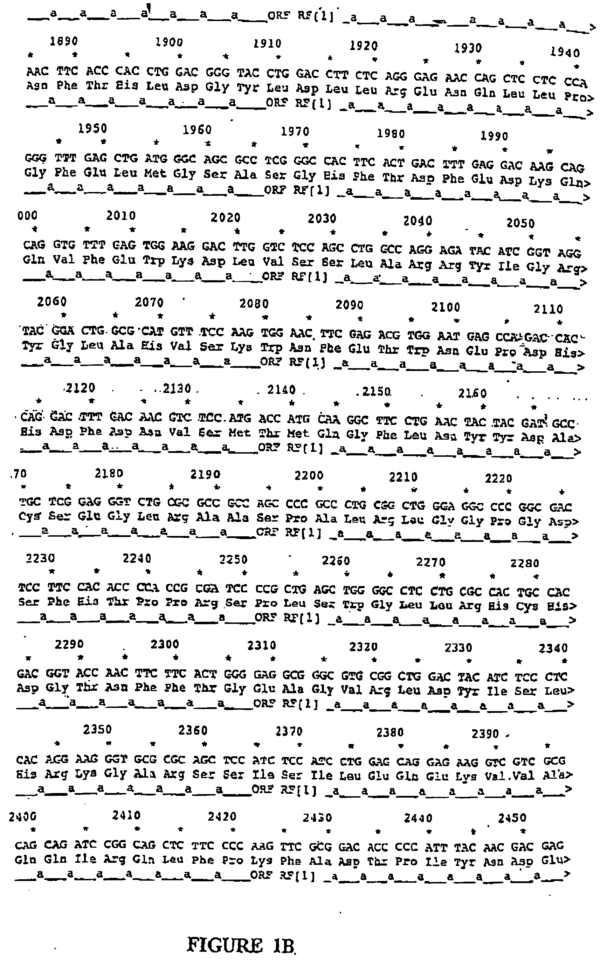

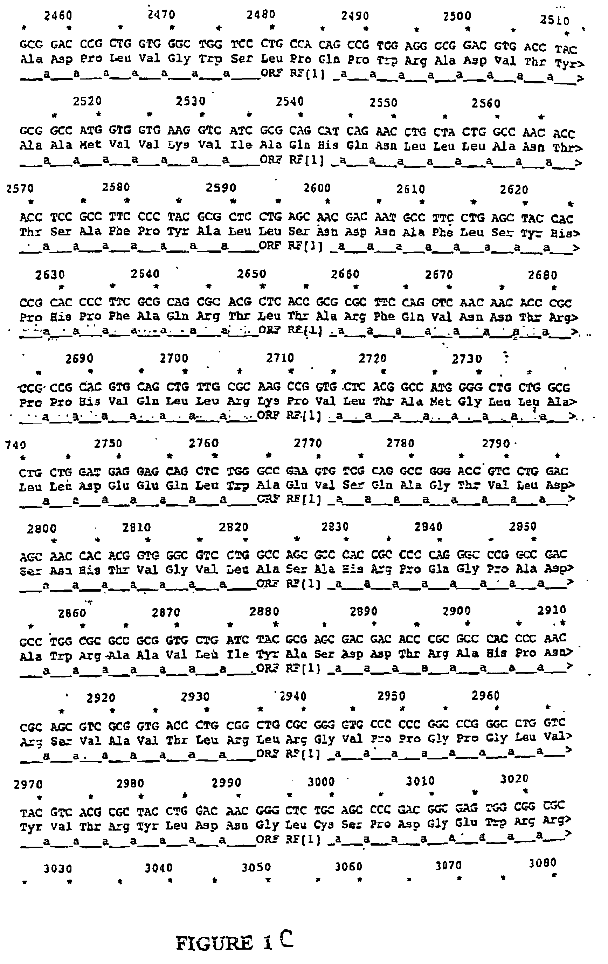

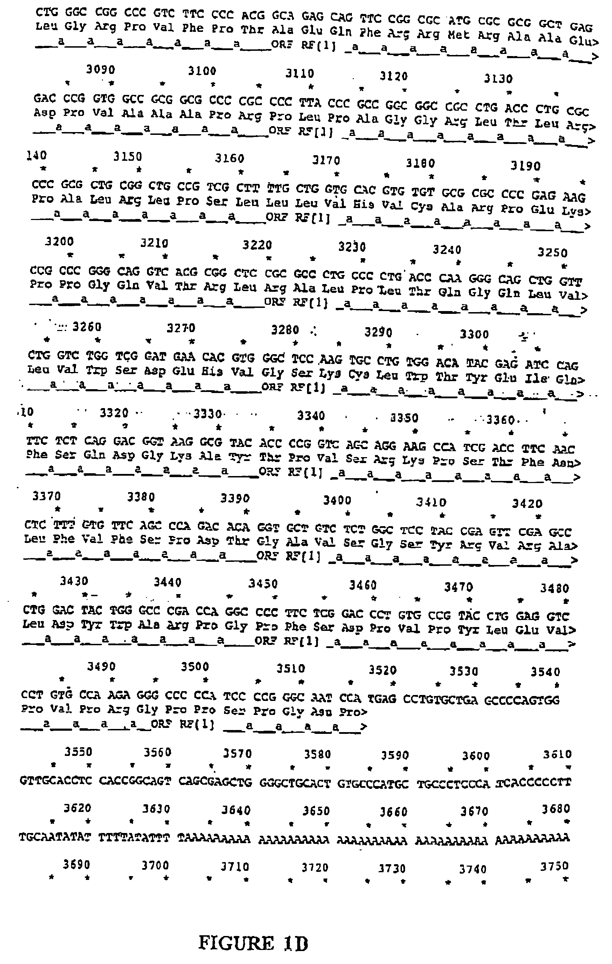

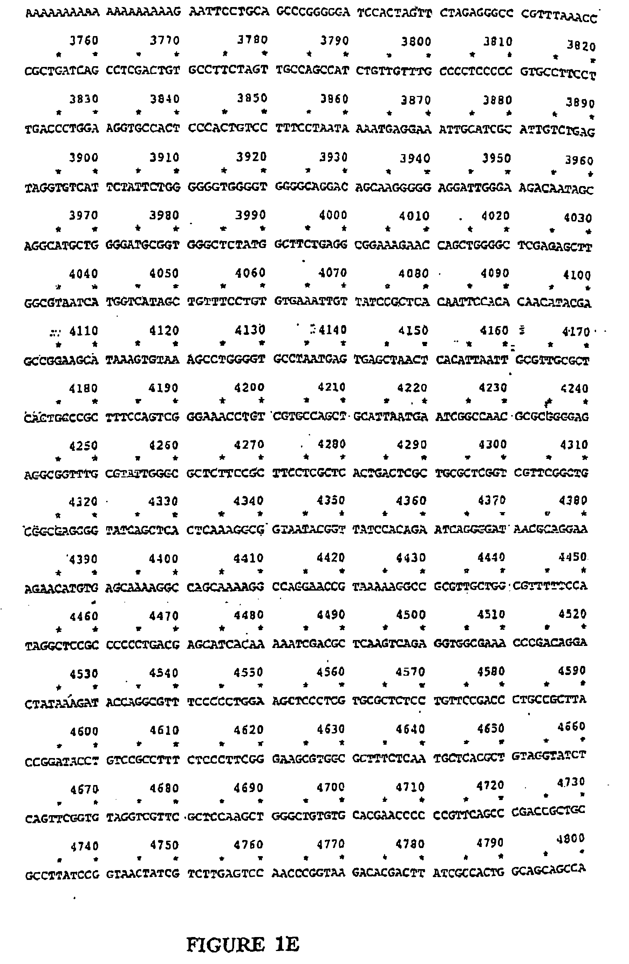

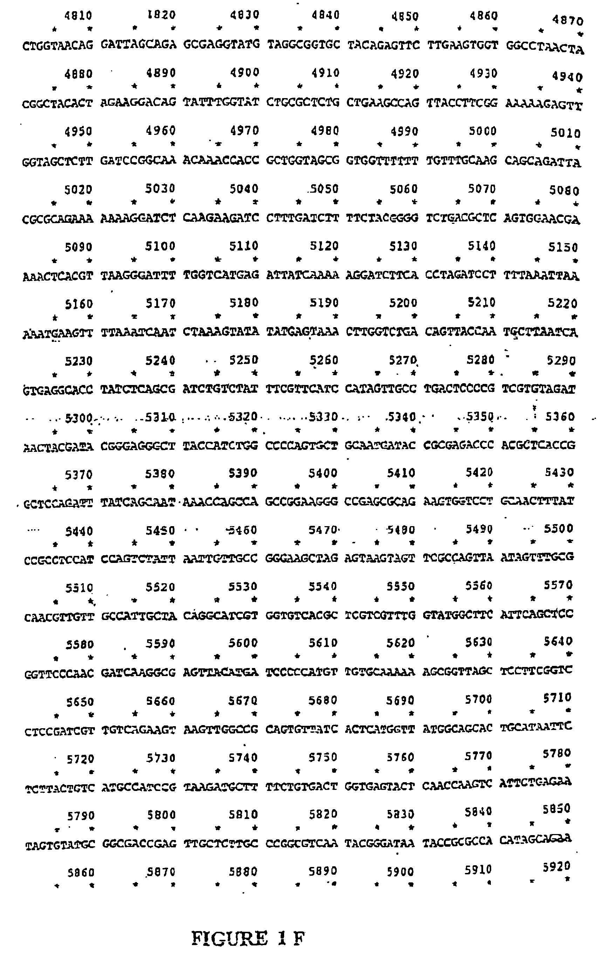

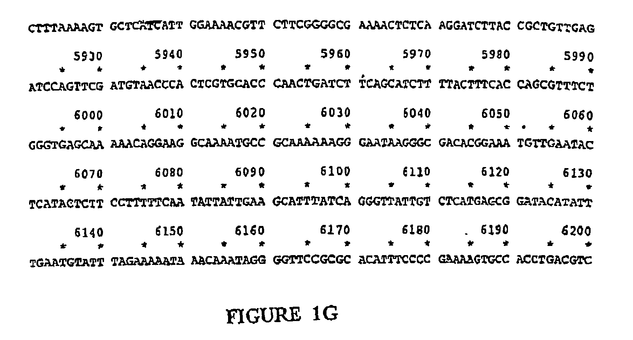

According to a first aspect of the present invention there is provided a recombinant human α-L-iduronidase enzyme, comprising amino acids 26 to 653 of Figure 1, with a purity or equal to or greater than 99%.

In one embodiment the recombinant human α-L-iduronidase enzyme is a high-uptake enzyme form which (a) comprises mannose-6-phosphate residues at positions 3 and 6 of the N-linked sugars and (b) exhibits an uptake affinity of less than 30 units of enzyme per ml or less than 2 nM.

In another embodiment the recombinant human α-L-iduronidase enzyme has a half-life inside cells of approximately five days.

In another embodiment the recombinant human α-L-iduronidase enzyme is substantially corrective for glycosaminoglycan storage disorders caused by iduronidase deficiency.

In another embodiment the recombinant human α-L-iduronidase enzyme has a specific activity greater than about 240,000 units per milligram protein.

According to a second aspect of the present invention there is provided a pharmaceutical composition comprising the recombinant human α-L-iduronidase enzyme in combination with a pharmaceutically suitable carrier.

In one embodiment the pharmaceutical composition comprises a sodium chloride solution, a buffer and polysorbate 80.

In another embodiment the recombinant human α-L-iduronidase enzyme is present in the pharmaceutical composition at a concentration of about 0.5 mg/mL and about 125,000 units per mL.

In one embodiment the sodium chloride solution is at a concentration of about 150 mM.

In another embodiment the buffer is a sodium phosphate buffer at a concentration of about 100 mM, and pH 5.4-5.9.

In another embodiment the pharmaceutical composition, after dilution into the dosage form, contains human albumen at a concentration of at least about 1 mg/mL.

In one embodiment, the pH of the solution is maintained at about 5.8

In one embodiment, the polysorbate 80 is maintained at 0.00 1%

The present invention features a method to mass produce human recombinant α-L-iduronidase in large scale amounts with appropriate purity to enable large scale production for long term patient use of the enzyme therapy. In a broad embodiment, the method comprises the step of transfecting a cDNA encoding for all or part of an α-L-iduronidase into a cell suitable for the expression thereof. In some embodiments, a cDNA encoding for a complete α-L-iduronidase is used, preferably a human α-L-iduronidase. However, in other embodiments, a cDNA encoding for a biologically active fragment or mutant thereof may be used. Specifically, one or more amino acid substitutions may be made while preserving or enhancing the biological activity of the enzymes. In other preferred embodiments, an expression vector is used to transfer the cDNA into a suitable cell or cell line for expression thereof. In one particularly preferred embodiment, the cDNA is transfected into a Chinese hamster ovary cell to create cell line 2.131. In yet other preferred embodiments, the production procedure features one or more of the following characteristics which have demonstrated particularly high production levels: (a) the pH of the cell growth culture may be lowered to about 6.5 to 7.0, preferably to about 6.8 - 7.0 during the production process, (b) as many as 2 to 3.5 culture volumes of the medium may be changed during each 24-hour period by continuous perfusion, (c) oxygen saturation may be optimized to about 40% but may be as high as 80%, (d) macroporous cellulose microcarriers with about 5% serum in the medium initially, may be used to produce cell mass followed by a rapid washout shift to protein-free medium for production, (e) a protein-free or low protein-medium such as a JRH Biosciences PF-CHO product may be optimized to include supplemental amounts of one or more ingredients selected from the group consisting of: glutamate, aspartate, glycine, ribonucleosides, and deoxyribonucleosides; (f) a stirred tank suspension culture may be perfused in a continuous process to produce iduronidase.

There is described a transfected cell line which features the ability to produce α-L-iduronidase in amounts which enable using the enzyme therapeutically. In preferred embodiments, the present invention features a recombinant Chinese hamster ovary cell line such as the 2.131 cell line that stably and reliably produces amounts of α-L-iduronidase which enable using the enzyme therapeutically. In some preferred embodiments, the cell line may contain more than 1 copy of an expression construct. In even more preferred embodiments, the cell line expresses recombinant α-L-iduronidase in amounts of at least 20 microgram per 10⁷ cells per day.

There is described vectors suitable to produce α-L-iduronidase in amounts which enable using the enzyme therapeutically. In preferred embodiments, the present invention features an expression vector comprising a cytomegalovirus promoter/enhancer element, a 5' intron consisting of a murine Ca intron, a cDNA encoding all or a fragment or mutant of an α-L-iduronidase, and a 3' bovine growth hormone polyadenylation site. Also, preferably the cDNA encoding all or a fragment or mutant of an α-L-iduronidase is about 2.2 kb in length. This expression vector may be transfected at, for example, a 50 to 1 ratio with any appropriate common selection vector such as pSV2NEO, to enhance multiple copy insertions. Alternatively, gene amplification may be used to induce multiple copy insertions.

The present invention provides novel α-L-iduronidase produced in accordance with the methods of the present invention and thereby present in amounts which enable using the enzyme therapeutically. The specific activity of the α-L-iduronidase according to the present invention is in excess of 200,000 units per milligram protein. Preferably, it is in excess of about 240,000 units per milligram protein. The molecular weight of the α-L-iduronidase of the present invention is about 82,000 daltons, about 70,000 daltons being amino acid, and about 12,000 daltons being carbohydrates.

There is described a novel method to purify α-L-iduronidase. According to a first embodiment, a cell mass may be grown in about 5% serum-containing medium, followed by a switch to a modified protein-free production medium without any significant adaptation to produce a high specific activity starting material for purification. In one preferred embodiment, a three step column chromatography may be used to purify the enzyme. Such a three step column chromatography may include using a blue sepharose FF, a Cu++ chelating sepharose chromatography and a phenyl sepharose HP chromatography. In another preferred embodiment, an acid pH treatment step is used to inactivate potential viruses without harming the enzyme. Concanavalin A-Sepharose, Heparin-Sepharose and Sephacryl 200 columns are removed and Blue-Sepharose and copper chelating columns added to increase the capacity of the large scale purification process, to reduce undesirable leachables inappropriate for long term patient use, and to improve the purity of the product.

There is described methods of treating diseases caused all or in part by a deficiency in α-L-iduronidase. In one embodiment, this method features administering a recombinant α-L-iduronidase or a biologically active fragment or mutant thereof alone or in combination with a pharmaceutically suitable carrier. In other embodiments, this method features transferring a nucleic acid encoding all or a part of an α-L-iduronidase into one or more host cells *in vivo*. Preferred embodiments include optimizing the dosage to the needs of the organism to be treated, preferably mammals or humans, to effectively ameliorate the disease symptoms. In preferred embodiments, the disease is Mucopolysaccharidosis I (MPS I), Hurler syndrome, Hurler-Scheie syndrome or Scheie syndrome.

There is described pharmaceutical compositions comprising a-L-iduronidase useful for treating a disease caused all or in part by a deficiency in α-L-iduronidase. Such compositions may be suitable for administration in a number of ways such as parenteral, topical, intranasal, inhalation or oral administration. Within the scope of this aspect are embodiments featuring nucleic acid sequences encoding all or a part of an α-L-iduronidase which may be administered *in vivo* into cells affected with an α-L-iduronidase deficiency.

### DESCRIPTION OF THE FIGURES

FIGURE 1 represents the nucleotide and deduced amino acid sequences of cDNA encoding α-L-iduronidase (SEQ ID NOs:1 and 2). Nucleotides 1 through 6200 are provided. Amino acids are provided starting with the first methionine in the open reading frame.

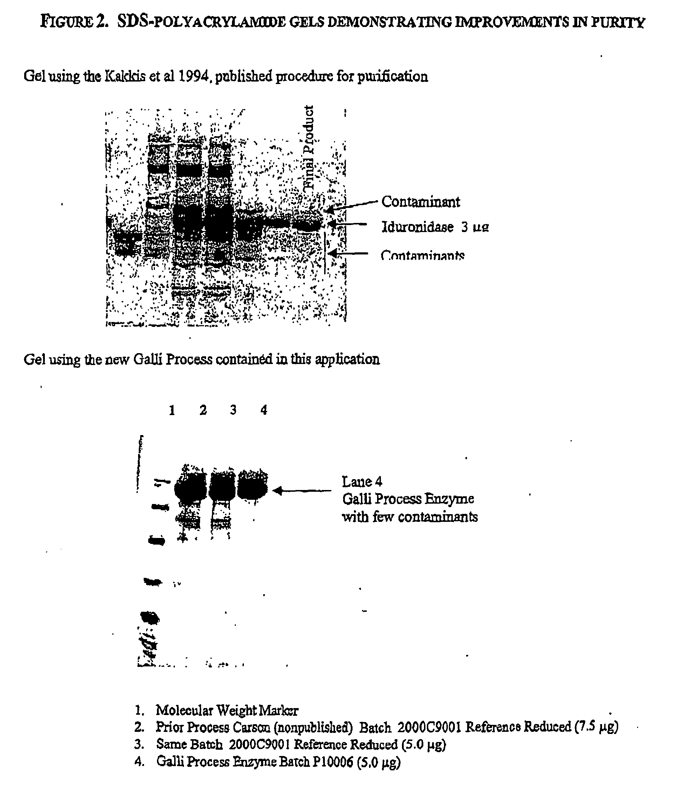

FIGURE 2 represents the results from SDS-PAGE runs of eluate obtained according to the procedures as described below. The top panel shows the SDS-PAGE results of purified α-L-iduronidase (3 micrograms) and contaminants from the production/purification scheme disclosed in Kakkis, *et al., Protein Expr. Purif*. 5: 225-232 (1994). In the bottom panel, SDS-PAGB results of purified α-L-iduronidase with contaminants from an unpublished prior production/purification process (U.S. Patent Application Serial Nos. 09/078,209 and 09/170,977) referred to as the Carson method in Lanes 2 (7.5 microgram α-L-iduronidase) and Lane 3 (5.0 microgram α-L-iduronidase) are compared to that of the production/purification process of the present invention referred to as the Galli Process (Lane 4 5 micrograms α-L-iduronidase). Lane 1 contains the molecular weight marker. FIGURE 2 shows that the Galli production/purification method of the present invention yields a highly purified α-L-iduronidase product with fewer contaminants in comparison with prior production/purification schemes.

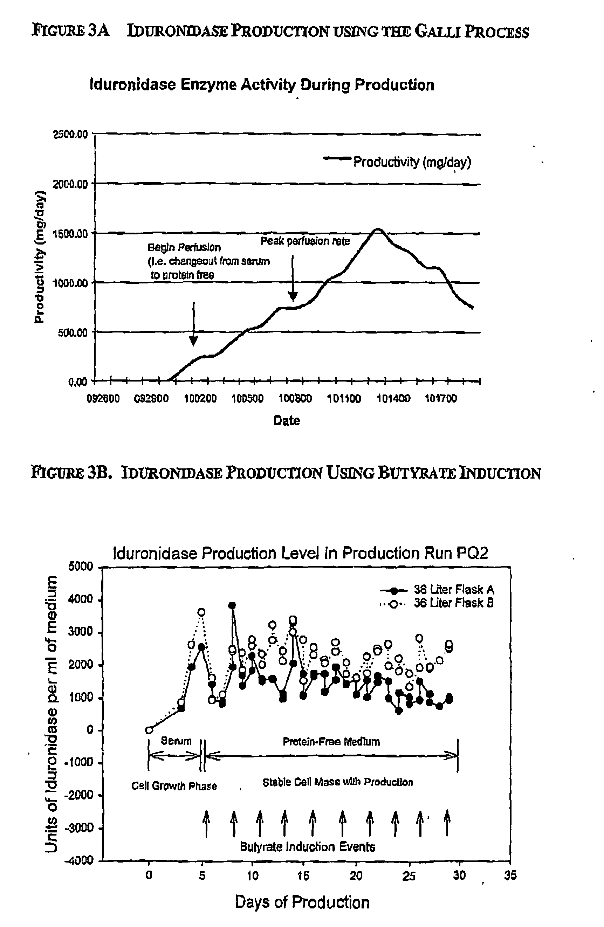

FIGURE 3 demonstrates the α-iduronidase production level over a 30-day period, during which time cells are switched at day 5 from a serum - containing medium to a serum-free medium. α-Iduronidase production was characterized by: (1) absence of a need for adaptation when cells are switched from serum-containing to serum-free medium at 100200 (top and bottom panels) with an uninterrupted increase in productivity (top panel); (2) a high level of production in excess of 4 mg per liter (1000 per mL) in a protein-free medium (bottom panel); and (3) a boost in α-iduronidase production with butyrate induction events (bottom panel).

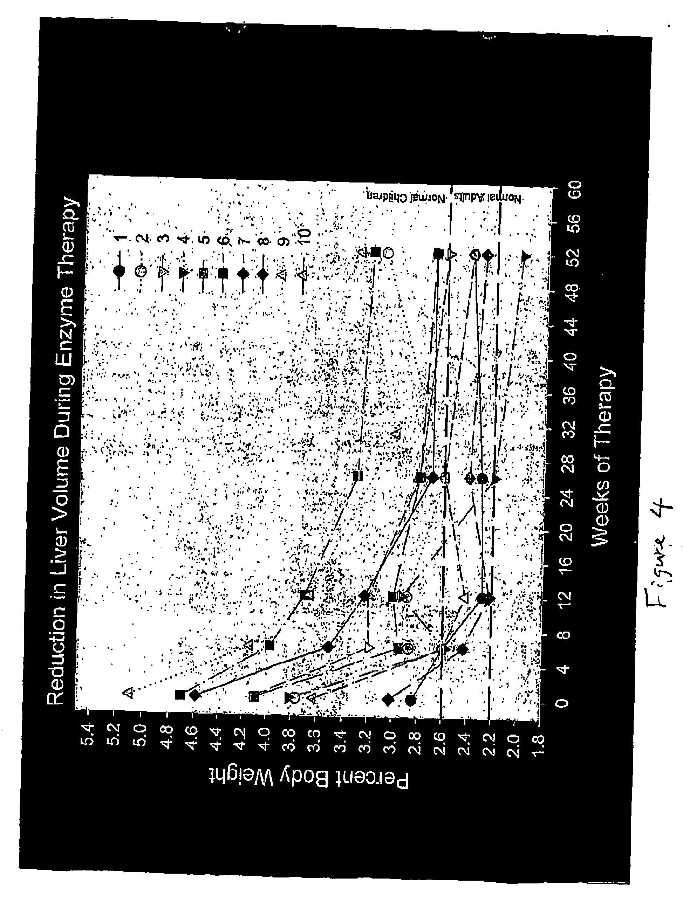

FIGURE 4 demonstrates a decrease in liver volume during enzyme therapy in MPS I patients.

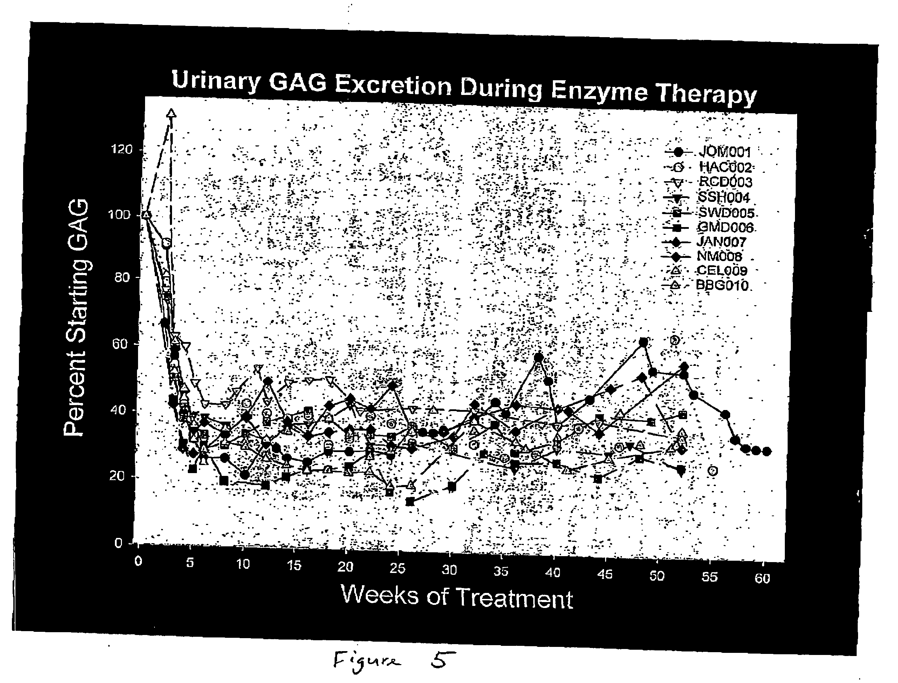

FIGURE 5 demonstrates urinary GAG excretion during enzyme therapy.

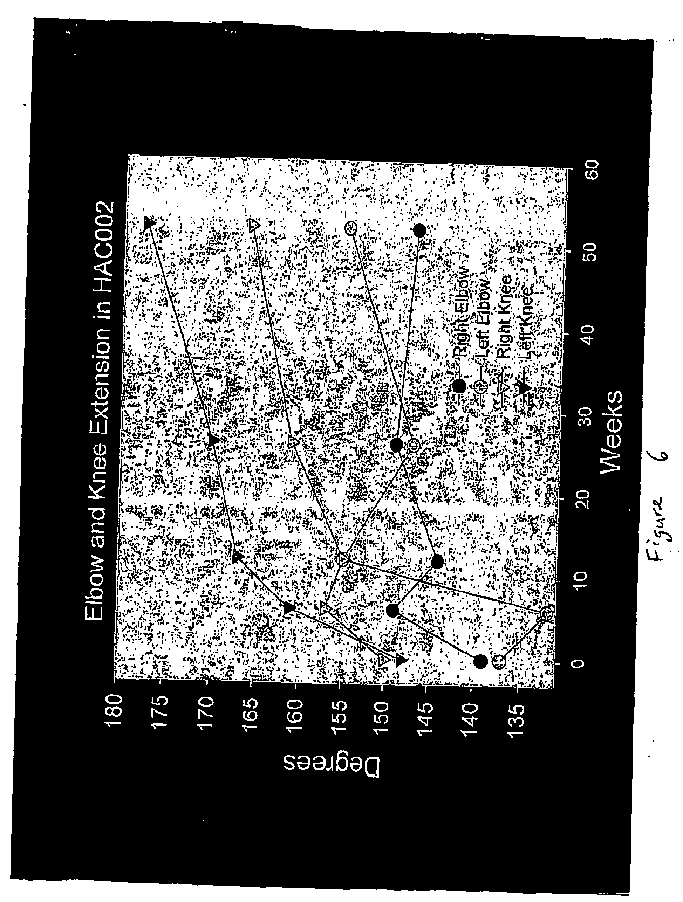

FIGURE 6 demonstrates elbow and knee extension in HAC002 during enzyme therapy.

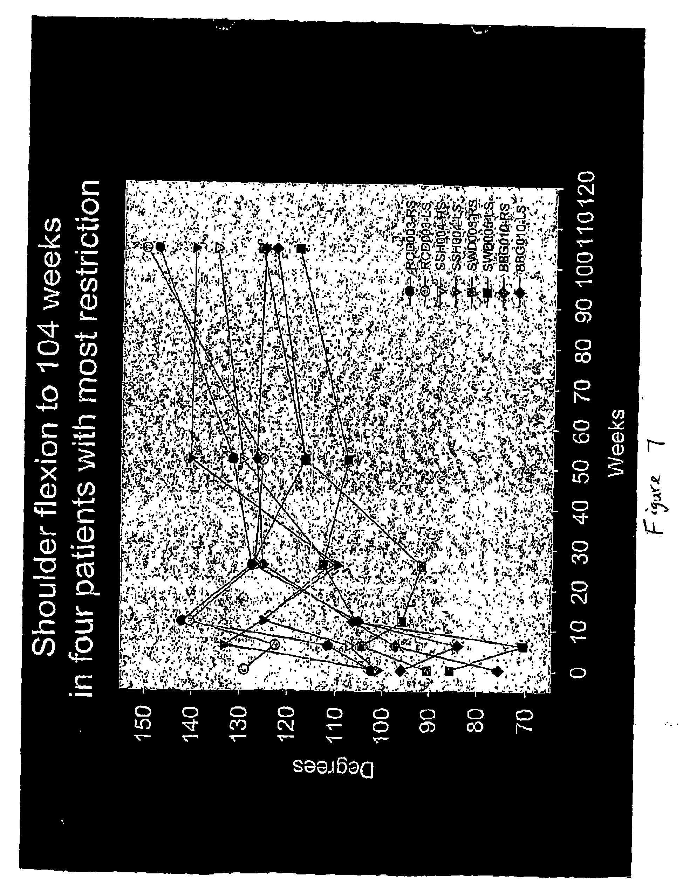

FIGURE 7 demonstrates shoulder flexion to 104 weeks in four patients with the most restriction during enzyme therapy.

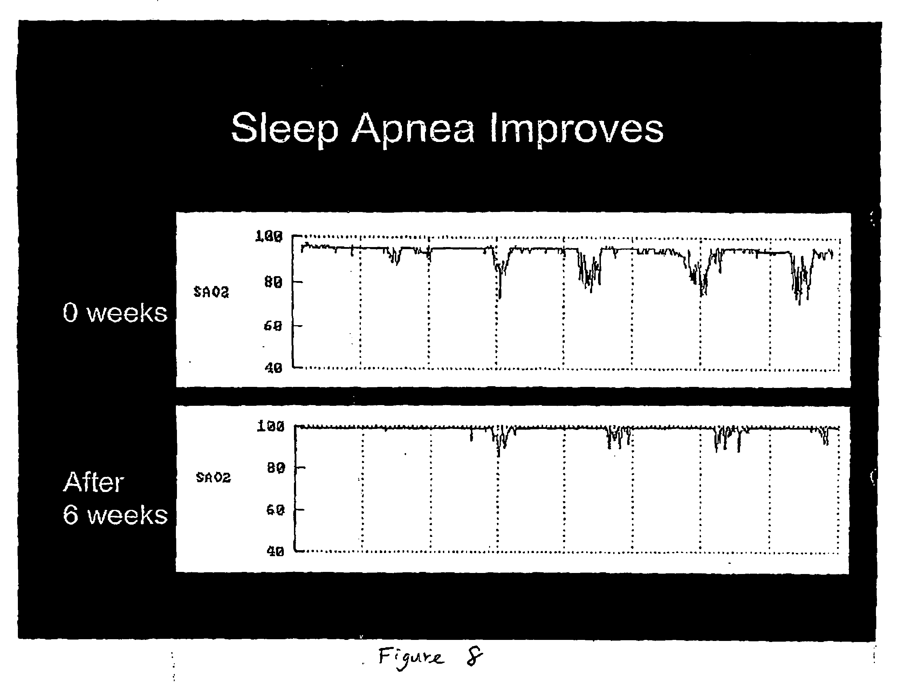

FIGURE 8 demonstrates improvement in sleep apnea before and after six weeks of therapy.

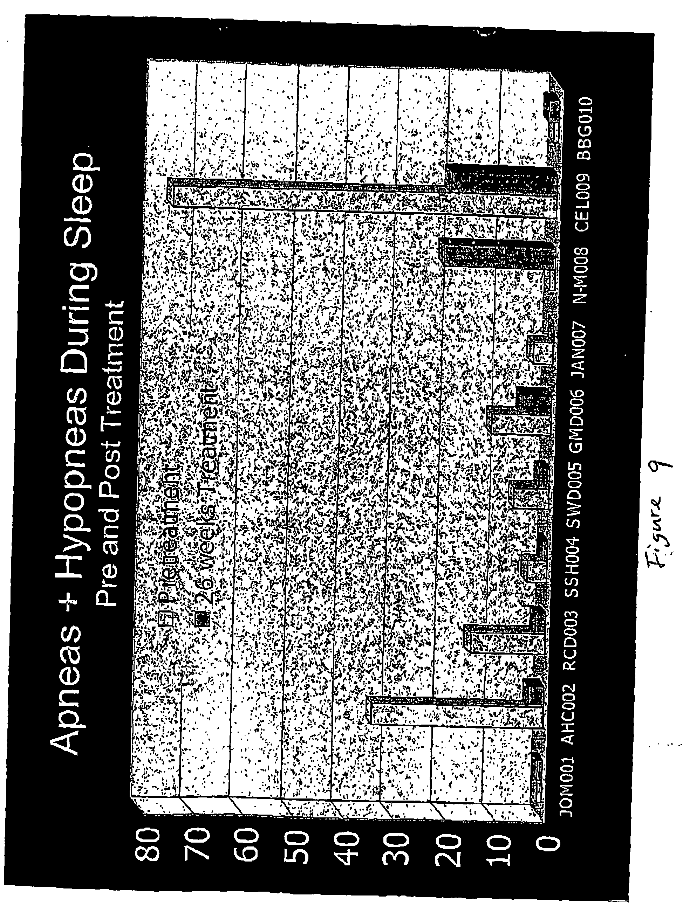

FIGURE 9 demonstrates the improvement in apneas and hypopneas during sleep with enzyme therapy in each individual patent.

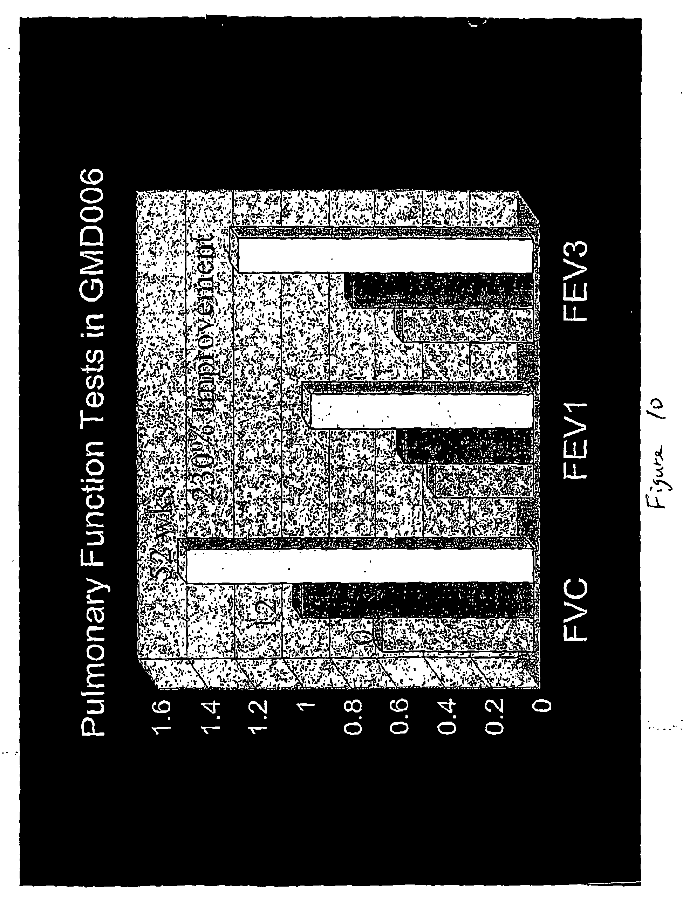

FIGURE 10 demonstrates the improvement in pulmonary function tests before and after 12 and 52 weeks of enzyme therapy in one patient.

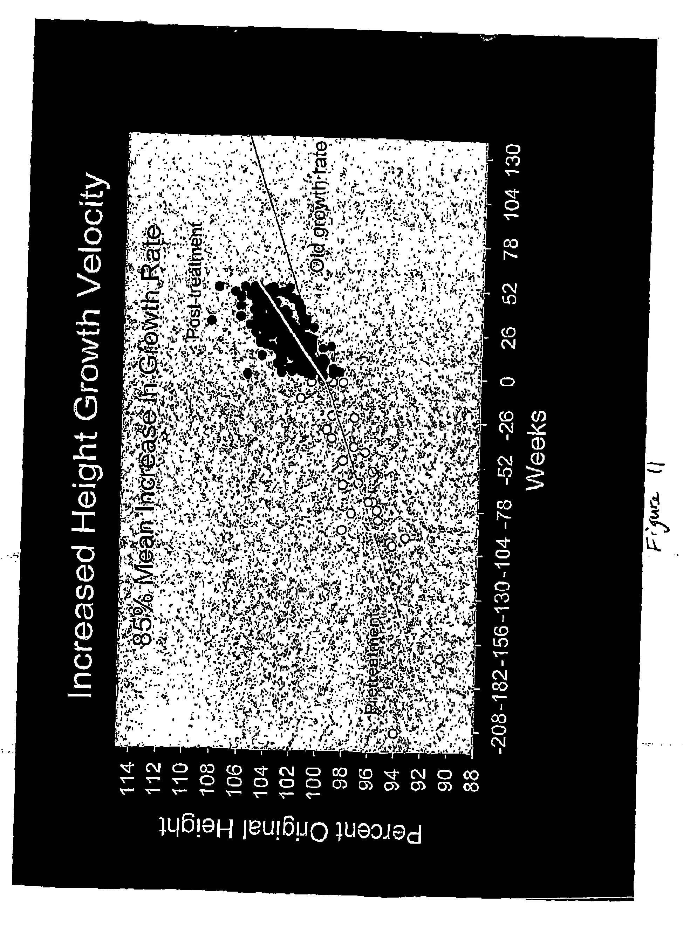

FIGURE 11 demonstrates increased height growth velocity with enzyme therapy.
FIGURE 12 shows the degree of contamination by Chinese Hamster Ovary Protein (CHOP) and degree of purity of α-L-iduronidase, produced by (1) the Carson method, an unpublished prior production/purification process (U.S. Patent Application Serial Nos. 09/078,209 and 09/170,977 and (2) the Galli method, the production/purification process of the present invention. Thus, FIGURE 12 shows that α-L-iduronidase produced and purified by the Galli method has a higher degree of purity and lower degree of CHOP contamination in comparison to that of the Carson method.

### DETAILED DESCRIPTION OF THE INVENTION

There is described a method to produce α-L-iduronidase in amounts which enable using the enzyme therapeutically. In general, the method features transforming a suitable cell line with the cDNA encoding for all of α-L-iduronidase or a biologically active fragment or mutant thereof. Those of skill in the art may prepare expression constructs other than those expressly described herein for optimal production of α-L-iduronidase in suitable cell lines transfected therewith. Moreover, skilled artisans may easily design fragments of cDNA encoding biologically active fragments and mutants of the naturally occurring α-L-iduronidase which possess the same or similar biological activity to the naturally occurring full-length enzyme.

To create a recombinant source for α-L-iduronidase, a large series of expression vectors may be constructed and tested for expression of a α-L-iduronidase cDNA Based on transient transfection experiments, as well as stable transfections, an expression construct may be identified that provides a particularly high level of expression. In one embodiment of the present invention, a Chinese hamster cell line 2.131 developed by transfection of the α-L-iduronidase expression construct and selection for a high expression clone provides particularly high level expression. Such a Chinese hamster cell line according to this embodiment of the present invention may secrete about 5,000 to 7,000 fold more α-L-iduronidase than normal. The α-L-iduronidase produced thereby may be properly processed, taken up into cells with high affinity and is connective for α-L-iduronidase deficient cells, such as those from patients suffering from Hurler's Syndrome.

The method for producing α-L-iduronidase in amounts that enable using the enzyme therapeutically features a production process specifically designed to mass produce commercial grade enzyme, wherein the quality of the enzyme has been deemed acceptable for administration to humans by regulatory authorities of various countries. The large scale production of commercial grade enzyme necessitates modifications of the cell culture scale, microcarrier systems, and purification scheme. In preferred embodiments, the cell culture scale is increased from 45 liters to 110 liters or more, with a change to continuous perfusion. The increase in scale is necessary to produce sufficient material for potential large scale production for long term patient use. According to preferred embodiments of such a process, microcarriers are used as a low cost scalable surface on which to grow adherent cells. In particularly preferred embodiments, such microcarriers are macroporous and are specifically composed of modified carbohydrates such as cellulose, e.g., Cytopore beads manufactured by Pharmacia. Macroporous cellulose microcarriers allow improved cell attachment and provide a larger surface area for attachment, which is expected to yield an increased cell density during the culture process. Higher cell densities are expected to increase productivity. In preferred embodiments, heparin-Sepharose and Sephacryl 200 columns are replaced with Blue-Sepharose and Copper chelating columns to increase the capacity of the large scale purification process and to improve the purity of the product. In a particularly preferred embodiment, the copper cheating column is used to reduce Chinese hamster ovary cell protein contaminants to very low levels appropriate for large scale distribution. Using embodiments of the present method featuring modifications and induction described below, approximately 15 mg per liter of culture per day, or more at peak culturing density can be produced starting with a 110 liter culture system.

According to other preferred embodiments of the method for producing α-L-iduronidase according to the present invention, a culture system, is optimized. In a first embodiment, the culture pH is lowered to about 6.5 to 7.0, preferably to about 6.7-7.0 during the production process. One advantage of such a pH is to enhance accumulation of lysosomal enzymes that are more stable at acidic pH. In a second embodiment, as many as 2 to 3.5 culture volumes of the medium may be changed during each 24-hour period by continuous perfusion. One advantage of this procedure is to enhance the secretion rate of recombinant α-L-iduronidase and to capture more active enzyme. In a third embodiment, oxygen saturation is optimized at about 40%. In a fourth embodiment, macroporous microcarriers with about 5% serum initially in the medium, are used to produce a cell mass followed by a rapid washout shift to a protein-free medium for production (FIGURE 3). In a fifth embodiment, a protein-free growth medium, such as a JRH Biosciences PF-CHO product, may be optimized to include supplemental amounts of one or more ingredients selected from the group consisting of: glutamate, aspartate, glycine, ribonucleosides and deoxyribonucleosides. In a sixth embodiment, as many as 2 to 3.5 culture volumes of the medium may be changed during each 24-hour period by continuous perfusion. Such an induction process may provide about a two-fold increase in production without significantly altering post-translational processing.

Particularly preferred embodiments of the method for producing α-L-iduronidase according to the present invention feature one, more than one, or all of the optimizations described herein and may be employed as described in more detail below. The production method of the present invention may, therefore, provide a production culture process having the following features:
1. A microcarrier based culture using macroporous microcarrier beads made of modified cellulose or an equivalent thereof is preferably used in large scale culture flasks with overhead stirring or an equivalent thereof. Attachment of cells to these beads may be achieved by culture in a 5% fetal bovine serum may be added to DME/F121:1 or a protein-free medium modified with ingredients including ribonucleosides, deoxyribonucleosides, pyruvate, non-essential amino acids, and HEPES. After about 3-6 days in this medium, a washout procedure is begun in which protein-free medium replaces the serum-containing medium at an increasing perfusion rate dependent on the glucose content and culture condition. Subsequently, and throughout the entire remaining culture period, the cells are cultivated in a protein-free medium. The use of a protein-free medium in enzyme production is beneficial in reducing the exposure risk of bovine spongiform encephalopathy (BSE) and other infectious biologic agents such as viruses to patients being treated with the enzyme, wherein the risk of BSE or other harmful agents is dependent on the amount of potential serum exposure. In prior published studies, the carriers used to grow the cells were bovine gelatin microcarriers, used at 1 gram per liter or 100 times the product concentration. Leaching of 1% of the gelatin protein from the microcarriers would represent a relative 100% contamination and thereby contribute to the risk of BSE. Thus, new carriers are either dextran or cellulose-based and consist of carbohydrates, and not animal-derived materials.
   FIGURE 3 shows that the cells are grown to a density in 5% serum containing medium and then switched without any adaptation to a protein-free medium. FIGURE 3 specifically shows that: 1) Cells survive and continue to produce iduronidase when shifted without adaptation. In contrast, other studies would suggest that adaptation to a protein-free medium is necessary. In the method of the present invention, enzyme production continues at levels comparable to serum containing medium. 2) α-L-iduronidase produced in a protein-free medium retains a level of production in excess of 4 mg per liter or 1,000 units per ml. 3) α-L-iduronidase produced in a protein-free medium has high uptake indicating that the shift in medium and, hence, a shift in carbohydrates being fed to cells, does not adversely effect the high uptake character of the enzyme. Eight lots of α-L-iduronidase have been produced and released in this manner with an uptake half maximal value of less than 2nM in all lots.
2. The culture conditions are preferably maintained at a dissolved oxygen of 40% of air saturation at a pH of about 6.8-7.0 and at a temperature of about 35-37° C. This may be achieved using a control unit, monitoring unit and appropriate probes such as those produced by Applikon® or Mettler®. However, skilled artisans will readily appreciate that this can easily be achieved by equivalent control systems produced by other manufacturers. An air saturation of about 40% results in improved α-L-iduronidase secretion though up to 80%% air saturation may be used. However, further increases in oxygen to, for example, 90% air saturation, do not provide significantly enhanced secretion over 80% air saturation. The dissolved oxygen may be supplied by intermittent or continuous oxygen sparging using a 5 micron stainless steel or larger opening sparger, or equivalent thereof. A pH of about 6.8-7.0 is optimal for the accumulation of the α-L-iduronidase enzyme. The enzyme is particularly unstable at pHs above about 7.0. Below a pH of about 6.7, the secretion rate may decrease, particularly below a pH of about 6.5. The culture is therefore maintained optimally between a pH of about 6.8-7.0 .
3. The production culture medium may be a modified form of the commercially available proprietary medium from JRH Biosciences called Excell PF CHO. This medium supports levels of secretion equivalent to that of serum using a cell line such as the 2.131 cell line. It may be preferably modified to include an acidic pH of about 6.8-7.0 (± 0.1), and buffered with HEPBS at 7.5 mM or 15 mM. The medium may contain 0.05 to 0.1% of Pluronics F-68 (BASIV), a non-ionic surfactant or an equivalent thereof which features the advantage of protecting cells from shear forces associated with sparging. The medium may further contain a proprietary supplement that is important in increasing the productivity of the medium over other protein-free media that are presently available. Those skilled in the art will readily understand that the choice of culture medium may be optimized continually according to particular commercial embodiments available at particular points in time. Such changes encompass no more than routine experimentation and are intended to be within the scope of the present invention.
4. The production medium may be analyzed using an amino acid analyzer comparing spent medium with starting medium. Such analyses have demonstrated that the 2.131 cell line depletes a standard PF CHO medium of glycine, glutamate and aspartate to a level of around 10% of the starting concentration. Supplementation of these amino acids to higher levels may result in enhanced culture density and productivity that may lead to a 2-3 fold higher production than at baseline. Skilled artisans will appreciate that other cell lines within the scope of the present invention may be equally useful for producing α-L-iduronidase according to the present method. Hence, more or less supplemental nutrients may be required to optimize the medium. Such optimizations are intended to be within the scope of the present invention and may be practiced without undue experimentation.
5. The medium may be supplemented with the four ribonucleosides and four deoxyribonucleosides each at about 10 mg/liter to support the dihydrofolate reductase deficient cell line 2.131. Skilled artisans will appreciate that other cell lines within the scope of the present invention may be equally useful for producing α-L-iduronidase according to the present method. Hence, more or less ribonucleosides and deoxyribonucleosides may be required to optimize the medium, and alternative sources of purines and pyrmidines for nucleic acid synthesis may be used such as hypoxanthine and thymidine. Such optimizations are intended within the scope of the present invention and may be practiced without undue experimentation.
6. After reaching confluence at about 3-6 days of culture, an increasing rate of continuous perfusion is initiated. A change of medium may be accomplished, for example, using a slant feed tube constructed and positioned to allow the uptake of medium without removal of the microcarriers even while the culture is stirred. By pumping out medium through the slant feed tube, microcarriers settle within the body of the tube inside the culture and are not removed from the culture during the change on medium. In this manner, the microcarriers with the cell mass are separated from supernatant containing the enzyme.
7. The rapid and frequent turnover of the medium has been shown by productivity studies to result in improved overall collection of enzyme from the cell culture. Less turnover of medium results in less total production of enzyme on a daily basis. Using the perfusion of 2-3.5 culture volumes per day, the cells may be maintained in excellent condition with high degrees of viability and a high level of productivity.
8. Production of α-L-iduronidase may be enhanced by the use of sodium butyrate induction of gene expression (FIGURE 3). Twenty lots of α-L-iduronidase were produced using butyrate induction at 2nM concentration with 2/3 washout every 12 hours after induction and reinduction every 48 hours for a 21-day production period. In Figure 3, the vertical arrows at the bottom indicate butyrate induction events. Each induction triggered a boost in α-L-iduronidase concentration in the medium.
   Systematic studies of a 2.131 cell line demonstrated that about 2 mM butyrate can be applied and result in about a two-fold or greater induction of enzyme production with minimal effects on carbohydrate processing. Lower levels of butyrate have not been shown to induce as well, and substantially higher levels may result in higher induction, but declining affinity of the produced enzyme for cells from patients suffering from α-L-iduronidase deficiency. Butyrate induction performed *in vitro* at 2mM for 24 hours or 5 mM, a more commonly used concentration resulted in uptakes in excess of 3 nM or 40 U/ml, or an average of three times the value observed in production lots. In addition, commonly used times of 24 hours or more and concentration of 5 mM were toxic to α-L-iduronidase producing cells and resulted in detachment and loss of cell mass.
   Results suggest that two-fold or greater induction results in less processing of the carbohydrates and less phosphate addition to the enzyme, as well as increasing toxicity. With respect to carbohydrate processing and the addition of phosphate groups, the importance of msunose-6-phosphate in enzyme replacement therapy is demonstrated by the observations that removal of the phosphate of two lysosomal enzymes, glucosidase and galactosamine 4-sulfatase leads to decreased uptake (Van der Ploeg, et al., J. Clin. Invest. 87: 513-518 (1991); Crawley, et al., J. Clin. Invest. 97: 1864-1873 (1996)). In addition, enzyme with low phosphate (Van Hose, et al., Proc. Natl. Acad. Sci. USA 93; 65-70 (1996) requires 1,000 units per ml for uptake experiments (nearly 100 times used for iduronidase) and effective doses in animal models require 14 mg/kg, or 28 times the dose used with high phosphate containing iduronidase (Kikuchi, et al.., J. Clin. Invest. 101: 827-833 (1998)).
   One particularly preferred aspect of the invention method uses 2 mM butyrate addition every 48 hours to the culture system. This embodiment results in about a two-fold induction of enzyme production using this method without significant effect on the uptake affinity of the enzyme (K-uptake of less than 30 U/ml or 2.0 mM).
9. There is described a transfected cell line, which possesses the unique ability to produce α-L-iduronidase in amounts, which enable using the enzyme therapeutically. In preferred embodiments, the present invention features a recombinant Chinese hamster ovary can line such as the 2.131 cell line that stably and reliably produces amounts of α-L-iduronidase. In preferred embodiments, the cell line may contain more than 1 copy of an expression construct comprising a CMV promoter, a Cα intron, a human α-L-iduronidase cDNA, and a bovine growth hormone polyadenylation sequence. In even more preferred embodiments, the cell line expresses α-L-iduronidase at amounts of at least about 20-40 micrograms per 10⁷ cells per day in a properly processed, high uptake form appropriate for enzyme replacement therapy. According to preferred embodiments of this aspect of the invention, the transfected cell line adapted to produce α-L-iduronidase in amounts which enable using the enzyme therapeutically, possesses one or more of the following features:
   1. The cell line of preferred embodiments is derived from a parent cell line wherein the cells are passaged in culture until they have acquired a smaller size and more rapid growth rate and until they readily attach to substrates.
   2. The cell line of preferred embodiments is transfected with an expression vector containing the cytomegalovirus promoter/enhancer element, a 5' intron consisting of the murine Cα intron between exons 2 and 3, a human cDNA of about 2.2 kb in length, and a 3' bovine growth hormone polyadenylation site. This expression vector may be transfected at, for example, a 50 to 1 ratio with any appropriate common selection vector such as pSV2NEO. The selection vector pSV2NEO in turn confers G418 resistance on successfully transfected cells. In particularly preferred embodiments, a ratio of about 50 to 1 is used since this ratio enhances the acquisition of multiple copy number inserts. According to one embodiment wherein the Chinese hamster ovary cell line 2.131 is provided, there is at least 1 copy of the expression vector for α-L-iduronidase. Such a cell line has demonstrated the ability to produce large quantities of human α-L-iduronidase (minimum 20 micrograms per 10 million cells per day). Particularly preferred embodiments such as the 2.131 cell line possess the ability to produce properly processed enzyme that contains N-linked oligosaccharides containing high mannose chains modified with phosphate at the 6 position in sufficient quantity to produce an enzyme with high affinity (K-uptake of less than 3 nM).
   3. The enzyme produced from the cell lines of the present invention such as a Chinese hamster ovary cell line 2.131 is rapidly assimilated into cells, eliminates glycosaminoglycan storage and has a half-life of about 5 days in cells from patients suffering from α-L-iduronidase deficiency.
   4. The cell line of preferred embodiments such as a 2.131 cell line adapts to large scale culture and stably produces human α-L-iduronidase under these conditions. The cells of preferred embodiments are able to grow and secrete α-L-iduronidase at the acid pH of about 6.6 to 7.0 at which enhanced accumulation of α-L-iduronidase can occur.
   5. Particularly preferred embodiments of the cell line according to the invention, such as a 2.131 cell line are able to secrete human α-L-iduronidase at levels exceeding 2,000 units per ml (8 micrograms per ml) harvested twice per day or exceeding 15 mg per liter of culture per day using a specially formulated protein-free medium.

There is described vectors suitable to produce α-L-iduronidase in amounts which enable using the enzyme therapeutically. The production of adequate quantities of recombinant α-L-iduronidase is a critical prerequisite for studies on the structure of the enzyme as well as for enzyme replacement therapy. The cell lines according to the present invention permit the production of significant quantities of recombinant α-L-iduronidase that is appropriately processed for uptake. Overexpression in Chinese hamster ovary (CHO) cells has been described for three other lysosomal enzymes, α-galactosidase (Ioamou, et al., J Cell. Biol. 119:1137-1150 (1992)), iduronate 2-sulfatase (Bielicki, et al., Biochem. J. 289: 241-246 (1993)), and N-acetylgalactosamine 4 -sulfatase (Amson, et al., Biochem. J. 284:789-794 (1992)), using a variety of promoters and, in one case, amplification. The present invention features a dihydrofolate reductase-deficient CHO cell line, but according to preferred embodiments of the invention amplification is unnecessary. Additionally, the present invention provides a high level of expression of the human α-L-iduronidase using the CMV immediate early gene promoter/enbancer.

The present invention features in preferred embodiments, an expression vector comprising a cytomegalovirus promoter/enhancer element a 5' intron consisting of the murine Cα intron derived from the murine long chain immunoglobulin Cα gene between exons 2 and 3, a human cDNA of about 2.2 kb in length, and a 3' bovine growth hormone polyadenylation site. This expression vector may be transfected at, for example, a 50 to 1 ratio with any appropriate common selection vector such as pSV2NEO. The selection vector such as pSV2NEO in turn confers G418 resistance on successfully transfected cells. In particularly preferred embodiments, a ratio of about 50 to 1 expression vector to selection vector is used since this ratio enhances the acquisition of multiple copy number inserts. According to one embodiment wherein the Chinese hamster ovary cell line 2.131 is provided, there are approximately 10 copies of the expression vector for α-L-iduronidase. Such an expression construct has demonstrated the ability to produce large quantities of human α-L-iduronidase (minimun 20 micrograms per 10 million cells per day) in a suitable cell line such as a Chinese Hamster ovary cell line 2.131. According to one aspect the present invention provides novel α-L-iduronidase produced in accordance with the methods of the present invention and thereby present in amounts that enable using the enzyme therapeutically. The methods of the present invention produce a substantially pure α-L-iduronidase that is properly processed and in high uptake form, appropriate for enzyme replacement therapy and effective in therapy *in vivo.*

The specific activity of the α-L-iduronidase According to the present invention is in excess of about 200,000 units per milligram protein. Preferably, it is in excess of about 240,000 units per milligram protein using the original assay methods for activity and protein concentration. A novel validated assay for the same enzyme with units expressed as micromoles per min demonstrates an activity of 100 units/ml (range of 70-130) and a protein concentration by absorbance at 280 nM of 0.7 mg/ml (0.6-0.8) with an average specific activity of 143 units per mg. The molecular weight of the full length α-L-iduronidase of the present invention is about 82,000 daltons comprising about 70,000 daltons of amino acids and 12,000 daltons of carbohydrates. The recombinant enzyme of the present invention is endocytosed even more efficiently than has been previously reported for a partially purified preparation of urinary enzyme. The recombinant enzyme according to the present invention is effective in reducing the accumulation of radioactive S-labeled GAG in α-L-iduronidase-deficient fibroblasts, indicating that it is transported to lysosomes, the site of GAG storage. The remarkably low concentration of α-L-iduronidase needed for such correction (half-maximal correction at 0.7 pM) may be very important for the success of enzyme replacement therapy.

The human cDNA of α-L-iduronidase predicts a protein of 653 amino acids and an expected molecular weight of 70,000 daltons after signal peptide cleavage. Amino acid sequencing reveals alanine 26 at the N-terminus giving an expected protein of 629 amino acids. Human recombinant ±-L-iduronidase has a Histidine at position 8 of the mature protein. The predicted protein sequence comprises six potential N-linked oligosaccharide modification sites. All of these may be modified in the recombinant protein. The third and sixth sites have been demonstrated to contain one or more mannose 6-phosphate residues responsible for high affinity uptake into cells. The following peptide corresponds to Amino Acids 26-45 of Human Recombinant α-L-iduronidase with an N-terminus alanine and the following sequence (SEQ. ID NO. 2): ala-glu-ala-pro-his-leu-val-his-val-asp-ala-ala-arg-ala-leu-trp-pro-leu-arg-arg

The overexpression of the α-L-iduronidase of the present invention does not result in generalized secretion of other lysosomal enzymes that are dependent on mannose-6-P targeting. The secreted recombinant α-L-iduronidase is similar to normal secreted enzyme in many respects. Its molecular size, found in various determinations to be 77, 82, 84, and 89 kDa, is comparable to 87 kDa, found for urinary corrective factor (Barton et al., J. Biol. Chem. 246: 7773-7779 (1971)), and to 76 kDa and 82 kDa, found for enzyme secreted by cultured human fibroblasts (Myerowitz, et al., J. Biol. Chem. 256: 3044-3048 (1991); Taylor, et al., Biochem. J 274:263-268 (1991)). The differences within and between the studies are attributed to imprecision of the measurements. The pattern of intracellular processing of the recombinant enzyme, a slow decrease in molecular size and the eventual appearance of an additional band smaller by 9 kDa is the same as for the human fibroblast enzyme. This faster band arises by proteolytic cleavage of 80 N-teminal amino acids.

The present invention features a novel method to purify α-L-iduronidase. In preferred embodiments, the present invention features a method to purify recombinant α-L-iduronidase that has been optimized to produce a rapid and efficient purification with validatible chromatography resins and easy load, wash and elute operation. The method of purifying α-L-iduronidase of the present invention involves a series of column chromatography steps, which allow the high yield purification of enzyme from protein-free production medium. Specifically, Concanavalin A-Sepharose, Heparin-Sepharose and Sephacryl 200 columns were replaced with Blue-Sepharose and Copper chelating columns to increase the capacity of a large-scale purification process, to reduce leachables and to improve the purity of the product. Concanavalin A lectin is often used to bind enzyme in an initial purification step in the prior published study, and is a protein lectin derived from plants. Concanavalin A is known to leach from columns and contaminate lysosomal enzyme preparations. Such leaching could cause activation of T cells in treated patients and hence is deemed inappropriate for human administration (Furbish, et al., Proc. Natl. Acad. Sci. USA 74: 3560-3563 (1977)). Thus, the use of Concanavalin A is avoided in the present purification scheme. In a prior study, the human liver α-L-iduronidase could not be recovered from phenyl columns without high concentrations of detergent (1% Triton X100) denaturation. Hence, a phenyl column was not used in a published purification scheme of this enzyme (Clements, et al., Eur. J. Biochem, 152: 21-28 (1985). The endogenous human liver enzyme is highly modified within the lysosomes by hydrolases which remove sialic acid and phosphate residues and proteases which nick the enzyme. In contrast, the overexpression of recombinant α-L-iduronidase causes 50% of the enzyme to be secreted rather than transported to the lysosome (Zhao, et al., J. Biol. Chem. 272: 22758-22765 (1997), Hence, recombinant iduronidase will have a full array of sialic acid and phosphate residues, which lead to a higher degree of water solubility and lower affinity to the phenyl column. The increased hydrophilicity allows the enzyme to be eluted under non-denaturing conditions using the low salt solutions of around 150-700 mM NaCl. This feature of the recombinant enzyme allows it to be purified in large scale without the use of detergents.

Recombinant α-L-iduronidase over-expressed in a Chinese Hamster Ovary (CHO) cell line, has been purified to near homogeneity following a 3-step column chromatography process. The first column involves an affinity chromatography step using Blue Sepharose 6 FF. The Blue Sepharose 6 FF eluate is then further purified by another affinity chromatography step using Cu⁺⁺ Chelating Sepharose FF. The final polish of the highly purified enzyme is achieved by hydrophobic interaction chromatography using Phenyl Sepharose High Performance (HP). The over-all yield ranges from 45 to 55 percent and the purity of the final product is > 99%. The process is robust, reproducible, and scalable for large-scale manufacturing. The purified enzyme has been characterized with respect to its enzymatic activity using a fluorescence-based substrate, and its functional uptake by fibroblast cells. The enzyme has also been characterized for substrate specificity, carbohydrate profiles, and isoelectric focusing (IEF) profiles.

Particularly preferred embodiments of the method for purifying α-L-iduronidase according to the present invention feature more than one or all of the optimizations according to the following particular embodiments. The purification method of the present invention may therefore provide a purified α-L-iduronidase having the characteristics described herein.
1. pH Adjustment/Filtration: The pH of filtered harvest fluid (HF) is adjusted to 5.3 with 1 M H₃PO₄ and then filtered through a 0.45 µ filter (e.g. Sartoclean, Sartorius).
2. Blue Sepharose FF chromatography: This affinity chromatography step serves to capture iduronidase to reduce the volume and to purify iduronidase by approximately seven to ten fold.

| | |
|---|---|
| Loading capacity: | 4 mg/ml (total protein per ml of resin) |
| Equilibration buffer: | 10 mM NaPO₄, pH 5.3 |
| Wash buffer: | 400 mM NaCl, 10 mM NaPO₄, pH 5.3 |
| Elution buffer: | 0.8 M NaCl, 10 mM NaPO₄, pH 5.3 |
| Regeneration buffer: | 2 M NaCl, 10 mM NaPO₄, pH 5.3 |
| Fold of purification: | 7-10 |
| Yield: | 70-85% |

3. Cu⁺⁺ Cheating Sepharose FF chromatography: The Cu⁺⁺ Chelating affinity chromatography step is very effective for removing some contaminating CHO proteins. The inclusion of 10% glycerol in all the buffers seems to be crucial for the quantitative recovery of iduronidase.

| | |
|---|---|
| Loading capacity: | 2 mg/ml |
| Equilibration buffer: | 1 M NaCl, 25 mM NaAc, pH 6.0, 10% Glycerol |
| Wash buffer: | 1 M NaCl, 25 mM NaAc, pH 4.0, 10% Glycerol |
| Elution buffer: | 1 M NaCl, 25 mM NaAc, pH 3.7, 10% Glycerol |
| Regeneration buffer: | 1 M NaCl, 50 mM EDTA, pH 8.0 |
| Fold of purification: | 2-5 |
| Yield: | 80% |

4. Phenyl Sephrose HP chromatography: phenyl Sephrose is used as the last step to further purify the product as well as to reduce residual leached Cibacron blue dye and Cu⁺⁺ ion carried over from previous columns.

| | |
|---|---|
| Loading capacity: | 1 mg/ml |
| Equilibration buffer: | 2 M NaCl, 10 mM NaPO₄, pH 5.7 |
| Wash buffer: | 1.5 M NaCl, 10 mM NaPO₄, pH 5.7 |
| Elution buffer: | 0.7 M NaCl, 10 mM NaPO₄, pH 5.7 |
| Regeneration buffer: | 0 M NaCl, 10 mM NaPO₄, pH 5.7 |
| Fold of purification: | 1.5 |
| Yield: | 90% |

5. Ultrafiltration (UF)/Diafiltration (DF)/Final formulation: The purified iduronidase is concentrated and diafiltered to a final concentration of 1 mg/ml in formulation buffer (150 mM NaCl, 100 mM NaPO₄, pH 5.8) using a tangential flow filtration (TFF) system (e.g. Sartocon Slice from Sartorius). The enzyme is then sterilized by filtering through a 0.2-micron filter (e.g., cellulose acetate or polysulfone) and filled into sterile vials.
6. Characterization of Purified Iduronidase: Analysis of enzyme purity using SDS-PAGE stained with Coomassie Blue or Silver and Western blot analysis. Analysis of enzymatic activity using 4MU-sulfate as substrate. Analysis of functional uptake using fibroblast cell assay. Analysis of carbohydrates by FACE. Analysis of IEF profiles.

Enzyme purified in this manner has been shown to contain mannose-6-phosphate residues of sufficient quantity at positions 3 and 6 of the N-linked sugars to give the enzyme uptake affinity of less than 30 units per ml (less than 2 nM) enzyme. The enzyme is substantially corrective for glycosaminoglycan storage disorders caused by iduronidase deficiency and has a halt-life inside cells of approximately 5 days.

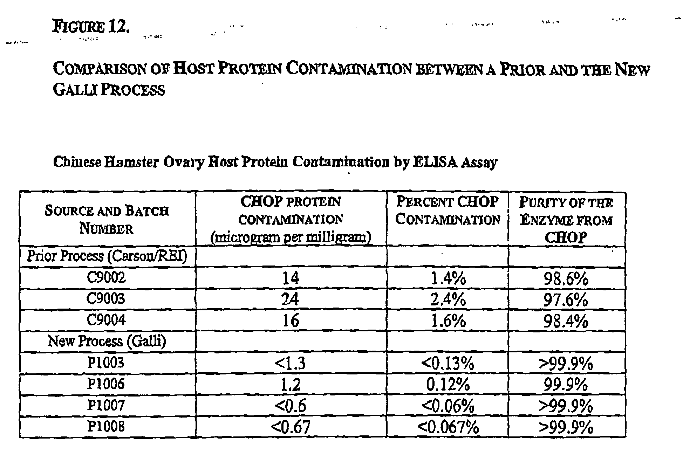

Prior α-L-iduronidase purification schemes (Kakkis, et al., Protein Expr. Purif. 5: 225-232 (1994); Kakkis, et al., Biochem. Mol. Med. 58: 156-167 (1996); U.S. Patent Application Nos. 09/078,209 and 09/170,977) produced degrees of purity between 90% and less than 99%, which is not optimal for long-term human administration (See FIGURE 12). (These and all other U.S. patents herein are specifically incorporated herein by reference in their entirety.) Treatment with human recombinant α-L-iduronidase with a minimum purity of 97% was associated with some clinical reactions, specifically hives in 5 patients, and complement activation in 4 patients. All patients demonstrated a reaction to a protein that is a trace contaminant to the α-L-iduronidase. (FIGURE 2) Because this protein exists in both the final product and in the serum-free blank CHO cell line supernatant, the extraneous protein most likely originates from the CHO cell. The common proteins that appear to be activating the clinical allergic response are approximately 60kDaltons and 50kDaltons respectively, which are too small to be recombinant human iduronidase. Four patients developed an immune reaction to α-L-iduronidase at least transiently as well as to the Chinese hamster ovary cell host proteins. It is clear that even though the enzyme used to treat patients is highly purified, the degree of purification is important in reducing the immune response to contaminants. FIGURE 2 (SDS-PAGE) and FIGURE 12 (CHOP assay) demonstrate that α-L-iduronidase produced and purified by the production/purification scheme of the present invention has a higher degree of purity and lower degree of CHOP contamination in comparison to that of prior methods of production/purification. Thus, a greater than 97% purity is adequate for patient use, higher levels of purity are desirable and preferable. As shown in FIGURE 12, the optimized purification scheme described above achieves a degree of purity that is greater than 99% and importantly reduces Chinese hamster ovary cell host proteins to less than 1 percent, as determined by the Chinese Hamster Ovary Protein (CHOP) assay.

There is described methods of treating diseases caused all or in part by a deficiency in α-L-iduronidase. Recombinant α-L-iduronidase provides enzyme replacement therapy in a canine model of MPS 1. This canine model is deficient in α-L-iduronidase due to a genetic mutation and is similar to human MPS 1. Purified, properly processed α-L-iduronidase was administered intravenously to 11 dogs. In those dogs treated with weekly doses of 25,000 to 125,000 units per kg for 0.5, 3, 6 or 13 months, the enzyme was taken up in a variety of tissues and decreased the lysosomal storage in many tissues. The long term treatment of the disease was associated with clinical improvement in demeanor, joint stiffness, coat and growth. Higher doses of therapy (125,000 units per kg per week) result in better efficacy, including normalization of urinary GAG excretion in addition to more rapid clinical improvement in demeanor, joint stiffness and coat.

Enzyme therapy at even small doses of 25,000 units (O.1 mg/kg/wk) resulted in significant enzyme distribution to some tissues and decreases in GAG storage. If continued for over 1 year, some clinical effects were evident in terms of increased activity, size and overall appearance of health. The therapy at this dose did not improve other tissues that are important sites for disease in this entity such as cartilage and brain. Higher doses of 125,000 units (0.5 mg/kg) given 5 times over two weeks demonstrate that improved tissue penetration can be achieved, and a therapeutic effect at the tissue level was accomplished in as little as 2 weeks. Studies at this increased dose have been completed in two dogs for 15 months. These MPS I dogs are showing significant clinical improvement and substantial decreases in urinary GAG excretion into the near normal range. Other than an immune reaction controlled by altered administration techniques, the enzyme therapy has not shown significant clinical or biochemical toxicity. Enzyme therapy at this higher weekly dose is effective at improving some clinical features of MPS I and decreasing storage without significant toxicity.

In a seventh aspect, the present invention features novel pharmaceutical compositions comprising human α-L-iduronidase useful for treating a deficiency in α-L-iduronidase. The recombinant enzyme may be administered in a number of ways such as parenteral, topical, intranasal, inhalation or oral administration. Another aspect of the invention is to provide for the administration of the enzyme by formulating it with a pharmaceutically acceptable carrier, which may be solid, semi-solid, liquid, or an ingestable capsule. Example of pharmaceutical compositions include tablets, drops such as nasal drops, compositions for topical application such as ointments, jellies, creams and suspensions, aerosols for inhalation, nasal spray, and liposomes. Usually the recombinant enzyme comprises between 0.01 and 99% or between 0.01 and 99% by weight of the composition, for example, between 0.01 and 20% for compositions intended for injection and between 0.1 and 50% for compositions intended for oral administration.

To produce pharmaceutical compositions in this form of dosage units for oral application containing a therapeutic enzyme, the enzyme may be mixed with a solid, pulverulent carrier, for example lactose saccharose, sorbitol, mannitol, a starch such as potato starch, corn starch, amylopectin, laminaria powder or citrus pulp powder, a cellulose derivative or gelatin and also may include lubricants such as magnesium or calcium stearate or a Carbowax® or other polyethylene glycol waxes and compressed to form tablets or cores for dragees. If dragees are required, the cores may be coated for example with concentrated sugar solutions which may contain gum arabic, talc and/or titanium dioxide, or alternatively with a film forming agent dissolved in easily volatile organic solvents or mixtures of organic solvents. Dyestuffs can be added to these coatings, for example, to distinguish between different contents of active substance. For the composition of soft gelatin capsules consisting of gelatin and, for example, glycerol as a plasticizer, or similar closed capsules, the active substance may be admixed with a Carbowax® or a suitable oil, *e*.*g*., sesame oil, olive oil, or arachis oil. Hard gelatin capsules may contain granulates of the active substance with solid, pulverulent carriers such as lactose, saccharose, sorbitol, mannitol, starches such as potato starch, corn starch or amylopectin, cellulose derivatives or gelatin, and may also include magnesium stearate or stearic acid as lubricants.

Therapeutic enzymes of the subject invention may also be administered parenterally such as by subcutaneous, intramuscular or intravenous injection or by sustained release subcutaneous implant. In subcutaneous, intramuscular and intravenous injection, the therapeutic enzyme (the active ingredient) maybe dissolved or dispersed in a liquid carrier vehicle. For parenteral administration, the active material may be suitably admixed with an acceptable vehicle, preferably of the vegetable oil variety such as peanut oil, cottonseed oil and the like. Other parenteral vehicles such as organic compositions using solketal, glycerol, formal, and aqueous parenteral formulations may also be used.

For parenteral application by injection, compositions may comprise an aqueous solution of a water soluble pharmaceutically acceptable salt of the active acids according to the invention, desirable in a concentration of 0.01-10%, and optionally also a stabilizing agent and/or buffer substances in aqueous solution. Dosage units of the solution may advantageously be enclosed in ampules.

When therapeutic enzymes are administered in the form of a subcutaneous implant, the compound is suspended or dissolved in a slowly dispersed material known to those skilled in the art, or administered in a device which slowly releases the active material through the use of a constant driving force such as an osmotic pump. In such cases, administration over an extended period of time is possible.

For topical application, the pharmaceutical compositions are suitably in the form of an ointment, gel, suspension, cream or the like. The amount of active substance may vary, for example, between 0.05- 20% by weight of the active substance. Such pharmaceutical compositions for topical application may be prepared in known manner by mixing the active substance with known carrier materials such as isopropanol, glycerol, paraffin, stearyl alcohol, polyethylene glycol, etc. The pharmaceutically acceptable carrier may also include a known chemical absorption promoter. Examples of absorption promoters are, *e*.*g*., dimethylacetamide (U.S. Patent No. 3,472,931), trichloro ethanol or trifluoroethanol (U.S. Patent No. 3,891,757), certain alcohols and mixtures thereof (British Patent No. 1,001,949). A carrier material for topical application to unbroken skin is also described in the British patent specification No. 1,464,975, which discloses a carrier material consisting of a solvent comprising 40-70% (v/v) isopropanol and 0-60% (v/v) glycerol, the balance, if any, being an inert constituent of a diluent not exceeding 40% of the total volume of solvent.

The dosage at which the therapeutic enzyme containing pharmaceutical compositions are administered may vary within a wide range and will depend on various factors such as the severity of the disease, the age of the patient, etc., and may have to be individually adjusted. A possible range for the amount of therapeutic enzyme which may be administered per day is about 0.1 mg to about 2000 mg or about 1 mg to about 2000 mg.

The pharmaceutical compositions containing the therapeutic enzyme may suitably be formulated so that they provide doses within these ranges, either as single dosage units or as multiple dosage units. In addition to containing a therapeutic enzyme (or therapeutic enzymes), the subject formulations may contain one or more substrates or cofactors for the reaction catalyzed by the therapeutic enzyme in the compositions. Therapeutic enzymes containing compositions may also contain more than one therapeutic enzyme.

The recombinant enzyme employed in the subject methods and compositions may also be administered by means of transforming patient cells with nucleic acids encoding the recombinant α-L-iduronidase. The nucleic acid sequence so encoded may be incorporated into a vector for transformation into cells of the subject to be treated. Preferred embodiments of such vectors are described herein. The vector may be designed so as to integrate into the chromosomes of the subject, *e*.*g*., retroviral vectors, or to replicate autonomously in the host cells. Vectors containing encoding α-L-iduronidase nucleotide sequences may be designed so as to provide for continuous or regulated expression of the enzyme. Additionally, the genetic vector encoding the enzyme may be designed so as to stably integrate into the cell genome or to only be present transiently. The general methodology of conventional genetic therapy may be applied to polynucleotide sequences encoding α-L-iduronidase. Conventional genetic therapy techniques have been extensively reviewed. (Friedman, Science 244:1275-1281 (1989); Ledley, J. Inherit. Metab. Dis. 13:587-616 (1990); Tososhev, et al., Curr Opinions Biotech. 1:55-61 (1990)).

A particularly preferred method of administering the recombinant enzyme is intravenously. A particularly referred composition comprises recombinant α-L-iduronidase, normal saline, phosphate buffer to maintain the pH at about 5.8 and human albumin. These ingredients may be provided in the following amounts:

| | |
|---|---|
| α-L-iduronidase | 0.05-0.2 mg/mL or 12,500-50,000 units per mL |
| Sodium chloride solution | 150 mM in an IV bag, 50-250 cc total volume |
| Sodium phosphate buffer | 10-50 mM, pH 5.8 |
| Human albumin | 1 mg/mL |

The invention having been described, the following examples are offered to illustrate the subject invention by way of illustration, not by way of limitation.

### EXAMPLE 1

### Producing Recombinant α-L-iduronidase

Standard techniques such as those described by Sambrook, et al (Molecular Cloning: A Laboratory Manual, 2nd ed., Cold Spring Harbor Laboratory, Cold Spring Harbor, N.Y. (1987)) may be used to clone cDNA encoding human α-Liduronidase. The human α-L-iduronidase cDNA previously cloned was subcloned into PRCCMV (InVitrogen) as a HindIII-XbaI fragment from a bluescript KS subclone. An intron cassette derived from the murine immunoglobulin Cot intron between exons 2 and 3 was constructed using PCR amplification of bases 788-1372 (Tucker, et al., Proc. Natl. Acad. Sci. USA 78: 7684-7688 (1991) of clone pRIR14.5 (Kakkis, et a/., Nucleic Acids Res. 16:7796 (1988)). The cassette included 136 bp of the 3' end of exon 2 and 242 bp of the 5' end of exon 3, which would remain in the properly spliced cDNA. No ATG sequences are present in the coding region of the intron cassette. The intron cassette was cloned into the HindIII site 5' of the α-L-iduronidase cDNA. The neo gene was deleted by digestion with Xhol followed by recircularizing the vector to make pCMVhldu.

One vial of the working cell bank is thawed and placed in three T225 flasks in DME/F12 or PF-CHO plus supplements, plus 5% FBS and 500 µg/ml G418. After 2-5 days, the cells are passaged using trypsin-EDTA to a 1-liter spinner flask in the same medium for 2-5 days. The cells are then transferred to two 3-liter spinner flasks for 2-5 days, followed by four 8-liter spinner flasks for 2-5 days. The inoculum from the 8-liter spinner flasks is added to two 110-liter Applikon® stirred tank bioreactors with an 80-90 liter working volume. Macroporous cellulose microcarriers are added at 2 grams per liter (160 gram), with PF-CHO or DME/F12 plus supplements, 5% FBS and 500 µg/ml of G418 at a final volume of 80-90 liters. The flask is stirred by an overhead drive with a marine impeller. The culture is monitored for agitation speed, temperature, DO and pH probes and controlled the Applikon® control system with a PC interface. The parameters are controlled at the set points or range, 35-37° C depending on culture conditions, 40% air saturation, and pH 6.95, using a heating blanket, oxygen sparger and base pump. The culture is incubated for 3-5 days at which time the culture is emerging from the log phase growth at 1-3 x 10⁶ cells per ml. Thereafter, perfusion is initiated at an increasing rate with PF-CHO medium (with custom modifications, JRH Biosciences). The first four days of collection (range of 3-5 days) are set aside as "washout." The collection thereafter is the beginning of the production run. Production continues with medium changes of as much as 2-3.5 culture volumes per day for 20-36 days. The culture may be extended for 40 days or longer. The culture is monitored for temperature, pH and DO on a continuous basis. The purification of the enzyme proceeds as described above. Collected production medium containing iduronidase is then acidified to pH 5.3, filtered through a 0.2-micron filter and purified using Blue-Sepharose chromatography. The purified enzyme from multiple rounds of Blue-Sepharose chromatography are then pooled and applied to a copper chelating column and eluted with glycerol in the buffer at a pH of 3.7. The enzyme is held at the acidic pH to inactivate potential viruses. The copper column eluate is then adjusted to pH 5.7 and 2 M NaCl and loaded on the phenyl Sepharose column. The enzyme is eluted at 0.7 M NaCl. The eluate is concentrated and diafiltered into a formulation buffer of 150 mM NaCl, 100 mM NaPO4, pH5.8. The enzyme is filtered through a 40 nM filter to remove potential viruses and the filtrate adjusted to 0.001 % polysorbate 80. The formulated enzyme is sterilely bulk filled into sterile polyethylene containers. The bulk enzyme is then filtered and filled into 5 cc Type 1 glass vials appropriate for injectable pharmaceuticals, stoppered and capped.

### EXAMPLE 2

For bioreactors using single cell suspension, the seed train is prepared as described above in EXAMPLE 1. Using a single cell suspension simplifies bioreactor preparation and inoculation The bioreactor is inoculated with cells in DMEM/F12 medium (25% of reactor volume) and JRH 325 modified (25% of reactor volume). Medium equal to 50% of the working reactor volume is added over 48 hours. Perfusion (and harvest) is started when cell density reaches 1.0 e⁶ and the perfusion medium is the same as described above.

### EXAMPLE 3

Short-term intravenous administration of purified human recombinant α-L-iduronidase to 9 MPS I dogs and 6 MPS I cats has shown significant uptake of an enzyme in a variety of tissues with an estimated 50% or more recovery in tissues 24 hours after a single dose. Although liver and spleen take up the largest amount of enzymes, and have the best pathologic improvement, improvements in pathology and glycosaminoglycan content has been observed in many, but not all tissues. In particular, the cartilage, brain and heart valve did not have significant improvement. Clinical improvement was observed in a single dog on long-term treatment for 13 months, but other studies have been limited to 6 months or less. All dogs, and most cats, that received recombinant human enzyme developed antibodies to the human product. The IgG antibodies are of the complement activating type (probable canine IgG equivalent). This phenomena is also observed in at least 13% of alglucerase-treated Gaucher patients. Proteinuria has been observed in one dog which may be related to immune complex disease. No other effects of the antibodies have been observed in the other treated animals. Specific toxicity was not observed and clinical laboratory studies (complete blood counts, electrolytes, BLJN/cteatinine, liver enzymes, urinalysis) have been otherwise normal.

Enzyme therapy at even small doses of 25,000 units (0.1 mg/kg/wk) resulted in significant enzyme distribution to some tissues and decreases in GAG storage. If continued for over 1 year, significant clinical effects of the therapy were evident in terms of activity, size and overall appearance of health. The therapy at this dose did not improve other tissues that are important sites for disease in this entity such as cartilage and brain. Higher doses of 125,000 units (0.5 mg/kg) given 5 times over two weeks demonstrate that improved tissue penetration can be achieved and a therapeutic effect at the tissue level was accomplished in as little as 2 weeks. Studies at this increased dose are ongoing in two dogs for six months to date. These MPS I dogs are showing significant clinical improvement and substantial decreases in urinary GAG excretion into the normal range. Other than an immune reaction controlled by altered administration techniques, the enzyme therapy has not shown significant clinical or biochemical toxicity. Enzyme therapy at this higher weekly dose is effective at improving some clinical features of MPS I and decreasing storage without significant toxicity.

The results of these various studies in MPS I dogs and one study in MPS I cats show that human recombinant α-L-iduronidase is safe. Although these same results provide significant rationale that this recombinant enzyme should be effective in treating α-L-iduronidase deficiency, they do not predict the clinical benefits or the potential immunological risks of enzyme therapy in humans.

### EXAMPLE 4

The human cDNA of α-L-iduronidase predicts a protein of 653 amino acids and an expected molecular weight of 70,000 daltons after signal peptide cleavage. Amino acid sequencing reveals alanine 26 at the N-terminus giving an expected protein of 629 amino acids. Human recombinant α-L-iduronidase has a Histidine at position 8 of the mature protein. The predicted protein sequence comprises six potential N-linked oligosaccharide modification sites. All of these sites are modified in the recombinant protein. The third and sixth sites have been demonstrated to contain one or more mannose 6-phosphate residues responsible for high affinity uptake into cells.

This peptide corresponds to Amino Acids 26-45 of Human Recombinant α-L-iduronidase with an N-terminus alanine and the following sequence (SEQ ID NO. 2);
ala-glu-ala-pro-his-leu-val-his-val-asp-ala-ala-arg-ala-leu-trp-pro-leu-arg-arg

The recombinant enzyme has an apparent molecular weight of 82,000 daltons on SDS-PAGE due to carbohydrate modifications. Purified human recombinant α-L-iduronidase has been sequenced by the UCLA Protein Sequencing facility. It is preferred to administer the recombinant enzyme intravenously. Human recombinant α-L-iduronidase was supplied for the clinical trial in 10 mL polypropylene vials at a concentration of 100,000-200,000 units per mL. The final dosage form of the enzyme used in the clinical trial includes human recombinant α-L-iduronidase, normal saline, and 100 mM phosphate buffer at pH 5.8. These are prepared in a bag of normal saline. Polysorbate 80 at a final concentration of 0.001 % was added to the formulation to stabilize the protein against shear, thereby avoiding precipitation in the final product vials.

**Final Vial Formulation Currently in Use**

| Component | Composition |
|---|---|
| α-L-iduronidase | Target to 0.7 mg/mL or 100 (new) units per mL |
| Sodium chloride solution | 150 mM |
| Sodium phosphate buffer | 100 mM, pH 5.8 |
| Polysorbate 80 | 0.001% |

**Final Dosage Form Used in the Treatment of Patients**

| Component | Composition |
|---|---|
| α-L-iduronidase produce | 5-12 fold dilution of vial concentration |
| Sodium chloride solution | 50 mM Sodium phosphate buffer 100-250 cc IV bag |
| Human albumin | 1 mg/ml |

### EXAMPLE 5

### Effects of Intravenous Administration of α-L-iduronidase in Patients with Mucopolysaccharidosis I

Based on studies of cloning of cDNA encoding α-L-iduronidase (Scott, et al., Proc. Natl. Acad. Sci. USA 88: 9695-99 (1991); Stoltzfus, et al., J. Biol. Chem. 267: 6570-75 (1992)) and animal studies showing effects of α-L-iduronidase to reduce lysosomal storage in many tissues (Shull, et al., Proc. Natl. Acad. Sci. USA 91: 12937-41 (1994); Kakkis, et al., Biochem. Mol. Med. 58: 156-67 (1996)), a 52-week study was conducted to assess the safety and clinical efficacy of intravenous administration of highly purified α-L-iduronidase in ten patients with mucopolysaccharidosis I (MPS I).

Recombinant human α-L-iduronidase was produced and purified to greater than 97-99%. Patients demonstrated typical clinical manifestations of the disorder and diagnosis was confirmed by biochemical determination of α-L-iduronidase deficiency in leukocytes.

Patients were given recombinant human α-L-iduronidase (diluted in normal saline with 0.1% human serum albumin) intravenously at a dose of 125,000 units per kg (using original assay and unit definition); 3,000 units per kg were given over the first hour, and 61,000 units per kg in each of the following two hours. The dose of 125,000 units per kg is equivalent to 100 SI units per kg using the new assay. The infusions were prolonged up to 4-6 hours in patients who had hypersensitivity reactions.

At baseline and at 6, 12, 26 and 52 weeks depending on the evaluation, the patients underwent examinations including history, physical examinations by specialists, echocardiography, EKG, MRI, polysomnography (weeks 0 and 26), skeletal survey (weeks 0, 26, 52), range of motion measurements, corneal photographs, and skin biopsy (week 0) to set up fibroblast cultures for enzyme determination and genotyping. Range of motion measurements were performed with a goniometer and the maximum active (patient initiated) range was recorded for each motion. Shoulder flexion is movement of the elbow interiorly from the side of the body and elbow and knee extension represent straightening of the joint. Degrees of restriction represent the difference between the normal maximum range of motion for age and the measured value. Polysomnography was performed according to American Thoracic Society guidelines and apneic events (cessation of oro-nasal airflow for 10 seconds or more), hypopneic events (decreased oro-nasal airflow of 50% or more with desaturation of 2% or more, or evidence of arousal), minutes below 89% oxygen saturation and total sleep time recorded among the standard measurements required. From these data an apnea/hypopnea index was calculated by dividing the total number of apneic and hypopneic events by the number of hours of sleep. Biochemical studies included measurement of enzyme activity in leukocytes and brushings of buccal mucosal, urinary glycosaminoglycan levels, and tests for serum antibodies to recombinant human α-L-iduronidase (ELISA and Western blot). Organ volumes were determined by analysis of MRI digital image data using Advantage Windows workstation software from General Electric. The organ volume was measured in milliliters and was converted to weight assuming a density of 1 gram per mL Urinary glycosaminoglycan excretion was assayed by an adaptation of a published method. Western blots and ELISA assays for antibodies to recombinant human a-L-iduronidase were performed by standard methods. Uronic acids and N-sulfate of urinary glycosaminoglycans were analyzed by the orcinol, carbazole and MBTH methods, and by electrophoretic separations.

All patients received weekly infusions of recombinant human α-L-iduronidase administered for 52 weeks. The mean activity of α-L-iduronidase in leukocytes was 0.04 units per mg before treatment and when measured on average 7 days after an infusion (i.e. immediately before the next infusion), 4.98 units per mg, or 15.0 percent of normal. Enzyme activity was not detectable in buccal brushings prior to treatment, but 7 days after infusions it reached a level of 1 percent of normal.

Liver volute decreased by 19 to 37 percent from baseline in 9 patients and 5 percent in one patient at 52 weeks; the mean decrease was 25.0 percent (n=10, P<0.001). By 26 weeks, liver size was normal for body weight and age in 8 patients (Figure 1). In 2 patients (patients 6 and 9) with the largest relative liver size at baseline, liver size was close to normal at 52 weeks (3.2 and 3.3 percent of body weight, respectively). Spleen size decreased in 8 patients by 13 to 42 percent from baseline (mean decrease of 20 percent in 10 patients, P<0.001).

Urinary glycosaminoglycan excretion declined rapidly by 3 to 4 weeks and by 8-12 weeks had fallen by 60-80 percent of baseline. At 52 weeks, the mean reduction was 63 percent (range 53-74; p<0.001). Eight often patients had a 75 percent or greater reduction of the baseline amount of urinary glycosaminoglycan in excess of the upper limit of normal for age. The results were confirmed by assay of uronic acids and N-sulfate (a test specific for heparan sulfate). Electrophoresis studies of urine detected a. significant reduction in heparan sulfate and dermatan sulfate excretion but some excess dermatan sulfate excretion persisted in all patients.

The mean height increased 6.0 cm (5.2 percent) in the 6 prepubertal patients (Table 2) and their mean height growth velocity increased from 2.8 cm/yr to 5.2 cm/yr during treatment (P=0.011). For all 10 patients, mean body weight increased 3.2 kg (8.8 percent) and the mean increase was 4.2 kg (17.1 percent) for the 6 prepubertal patients (Table 2). In these 6 patients, the mean pretreatment weight growth velocity increased from 1.7 kg per year to 3.8 kg per year during treatment (P=0.04).

Shoulder flexion (moving the elbow anteriorly) increased in 6 of the 8 subjects evaluated at baseline with a mean improvement for the right and left shoulders of 28° and 26°, respectively (P < 0.002; Figure 2). Elbow extension and knee extension increased by a mean of 7.0° (P <0.03) and 3.2° (P=0.10), respectively, in the 10 patients (Figure 2).

Analysis of the improvement in individual patients revealed that the most restricted joints had the greatest improvement. For example at baseline, patients 5, 9 and 10 could not flex their shoulders (move the elbow anteriorly) beyond 100°, which increased 21° to 51° after treatment. Similarly, patients 2 and 9 had a substantial increase in knee extension. The changes in range of motion were accompanied by patient-reported increases in physical activities such as being able to wash their hair, hold a hamburger normally, hang from monkey bars, and play sports better.

Seven patients had a decrease in apnea and hypopnea events from 155 to 60 per night upon treatment (a 61 percent decrease) with a change in mean apcea/hypopnea index (total number of events per hour) from 2.1 to 1.0. Three patients had clinically significant sleep apnea and all three improved during treatment. In patient 2, the apnea/hypopnea index decreased from 4.5 at baseline to 0.4 at 26 weeks and total time of oxygen desaturation decreased from 48 minutes to 1 minute per night Patient 6 required nightly continuous positive airway pressure therapy before treatment due to severe desaturation (61 minutes below 89 percent saturation with continuous positive airway pressure in 368 minutes of sleep), but by 52 weeks, the patient tolerated the sleep study without CPAP and desaturated below 89 percent for only 8 minutes during 332 minutes of sleep. Patient 9 had an apnea hypopnea index of 9.5 which decreased to 4.0 by 26 weeks. Patient 8 worsened with an apnea hypopnea index of 0.1 increasing to 3.1 at 26 weeks and 9.3 at 52 weeks for unclear reasons. Eight of ten patients or their families reported improved breathing, and 5 of 7 noted quieter nighttime breathing, improved sleep quality and decreased daytime somnolence.

New York Heart Association functional classification was determined by serial patient interviews. All 10 patients reported improvement by one or two classes but there was no significant objective data from echocardiographic studies to verify direct cardiac benefit. The improved functional scores may reflect improvements in other aspects of MPS I disease rather than cardiac function. Comparing baseline to 52 weeks of treatment, echocardiography demonstrated decreased tricuspid regurgitation or pulmonic regurgitation in 4 patients but two patients (patients 2 and 7) had worsening mitral regurgitation. At baseline, patient 6 had atrial flutter and clinical signs of cardiac failure including dyspnea at rest and peripheral edema. By 12 weeks, he had normal sinus rhythm with first degree block and his dyspnea at rest and pitting edema resolved.

All 10 patients reported a lack of endurance and limitations of daily activities before treatment but exercise tolerance was not formally tested. During treatment, all patients improved and by 26 weeks, many were able to walk more, run and play sports. Patients 3, 4 and 5 reported the resolution of severe incapacitating headaches after treatment for 6-12 weeks.

Several patients reported decreased photophobia or conjunctival irritation. Visual acuity improved in one patient (20/1000 to 20/200 in one eye) and modestly in 2 others.

The results of this study indicate that intravenous administration of the highly purified recombinant human α-L-iduronidase of the present invention results in clinical and biochemical improvement in patients with Mucopolysaccharidosis I. The normalization of liver size and near normalization of urinary glycosaminoglycan excretion is consistent with data from studies in dogs with Mucopolysaccharidosis I, which demonstrated clearance of storage in the liver and decreased urinary glycosaminoglycan excretion in as little as 2 weeks.

Hypersensitivity reactions to the infusions of recombinant human α-L-iduronidase were less severe than predicted from studies in dogs. Though important in some patients, recurrent urticaria was manageable with premedication and adjustments in infusion rate. Antibodies specific to α-L-iduronidase were detected in 4 patients with usually subclinical complement activation, and both the antibodies and complement activation declined with time. Similar IgG-mediated immune responses have been previously noted in patients with Gauche disease treat with glucocerebrosidase, although the events were more frequent in our patients. Mucopolysaccharidosis I patients with a null genotype may have a greater immune response than in these 10 patients, none ofwhom has a null.

Thus, recombinant human α-L-iduronidase can reduce lysosomal storage and ameliorates some aspects of clinical disease of Mucopolysaccharidosis I.

The invention, and the manner and process of making and using it, are now described in such full, clear, concise and exact terms as to enable any person skilled in the art to which it pertains, to make and use the same.

## Claims

1. A recombinant human α-L-iduronidase enzyme, comprising amino acids 26 to 653 of Figure 1, with a purity of equal to or greater than 99%.

2. The recombinant human α-L-iduronidase enzyme of claim 1 which is a high-uptake enzyme form which (a) comprises mannose-6-phosphate residues at positions 3 and 6 of the N-linked sugars and (b) exhibits an uptake affinity of less than 30 units of enzyme per ml or less than 2 nM.

3. The recombinant human α-L-iduronidase enzyme of claim 1 or claim 2 which has a half-life inside cells of approximately five days.

4. The recombinant human α-L-iduronidase enzyme of claim 3 which is substantially corrective for glycosaminoglycan storage disorders caused by iduronidase deficiency.

5. The recombinant human α-L-iduronidase enzyme of any one of claims 1 to 4 with a specific activity greater than about 240,000 units per milligram protein.

6. A pharmaceutical composition comprising the recombinant human α-L-iduronidase enzyme of any one of claims 1 to 5 in combination with a pharmaceutically suitable carrier.

7. The pharmaceutical composition of claim 6 further comprising a sodium chloride solution, a buffer and polysorbate 80.

8. The pharmaceutical composition of claim 6 or 7, wherein said recombinant human α-L-iduronidase enzyme is present at a concentration of about 0.5 mg/mL and about 125,000 units per mL.

9. The pharmaceutical composition of claim 7 or 8, wherein said sodium chloride solution is at a concentration of about 150 mM.

10. The pharmaceutical composition of any one of claims 7 to 9, wherein said buffer is a sodium phosphate buffer at a concentration of about 100 mM, and pH 5.4-5.9.

11. The pharmaceutical composition of any one of claims 7 to 10 wherein after dilution into the dosage form human albumin is present at a concentration of at least about 1 mg/mL.

12. The pharmaceutical composition of any one of claims 7 to 11, wherein the pH of said solution is maintained at about 5.8.

13. The pharmaceutical composition of any one of claims 7 to 12, wherein said polysorbate 80 is maintained at 0.001 %.

## Patentansprüche

1. Rekombinantes humanes α-L-Iduronidaseenzym, welches die Aminosäuren 26 bis 653 aus Figur 1 umfaßt, mit einer Reinheit gleich oder größer als 99%.

2. Rekombinantes humanes α-L-Iduronidaseenzym nach Anspruch 1, welches eine Enzymform mit hoher Aufnahme ist, die (a) Mannose-6-phosphat-Reste an den Positionen 3 und 6 der N-verknüpften Zucker umfaßt und (b) eine Aufnahmeaffinität von weniger als 30 Einheiten Enzym pro ml oder weniger als 2 nM zeigt.

3. Rekombinantes humanes α-L-Iduronidaseenzym nach Anspruch 1 oder Anspruch 2, welches innerhalb von Zellen eine Halbwertszeit von ungefähr fünf Tagen hat.

4. Rekombinantes humanes α-L-Iduronidaseenzym nach Anspruch 3, welches durch Iduronidasemangel verursachte Glycosaminoglycanspeicherstörungen im wesentlichen korrigiert.

5. Rekombinantes humanes α-L-Iduronidaseenzym nach einem der Ansprüche 1 bis 4 mit einer spezifischen Aktivität, die größer ist als etwa 240.000 Einheiten pro Milligramm Protein.

6. Pharmazeutische Zusammensetzung, welche das rekombinante α-L-Iduronidaseenzym nach einem der Ansprüche 1 bis 5 in Kombination mit einem pharmazeutisch geeigneten Träger umfaßt.

7. Pharmazeutische Zusammensetzung nach Anspruch 6, welche weiterhin eine Natriumchloridlösung, einen Puffer und Polysorbat 80 umfaßt.

8. Pharmazeutische Zusammensetzung nach Anspruch 6 oder 7, wobei das rekombinante humane α-L-Iduronidaseenzym in einer Konzentration von etwa 0,5 mg/ml und etwa 125.000 Einheiten pro ml vorliegt.

9. Pharmazeutische Zusammensetzung nach Anspruch 7 oder 8, wobei die Natriumchloridlösung eine Konzentration von etwa 150 mM hat.

10. Pharmazeutische Zusammensetzung nach einem der Ansprüche 7 bis 9, wobei der Puffer ein Natriumphosphatpuffer in einer Konzentration von etwa 100 mM ist und einen pH-Wert von 5,4-5,9 hat.

11. Pharmazeutische Zusammensetzung nach einem der Ansprüche 7 bis 10, wobei nach Verdünnen in die Dosierungsform humanes Albumin in einer Konzentration von wenigstens etwa 1 mg/ml vorliegt.

12. Pharmazeutische Zusammensetzung nach einem der Ansprüche 7 bis 11, wobei der pH-Wert der Lösung auf etwa 5,8 gehalten wird.

13. Pharmazeutische Zusammensetzung nach einem der Ansprüche 7 bis 12, wobei das Polysorbat 80 auf 0,001 % gehalten wird.

## Revendications

1. Enzyme α-L-iduronidase humaine recombinante, comprenant les amino-acides 26 à 653 de la Figure 1, ayant une pureté égale ou supérieure à 99 %.

2. Enzyme α-L-iduronidase humaine recombinante suivant la revendication 1, qui est une forme d'enzyme à taux élevé d'absorption qui (a) comprend des résidus mannose-6-phosphate aux positions 3 et 6 des sucres liés par N et (b) présente une affinité d'absorption inférieure à 30 unités d'enzyme par ml ou inférieure à 2 nM.

3. Enzyme α-L-iduronidase humaine recombinante suivant la revendication 1 ou la revendication 2, qui a une demi-vie à l'intérieur des cellules d'approximativement cinq jours.

4. Enzyme α-L-iduronidase humaine recombinante suivant la revendication 3, qui présente une activité de correction substantielle pour des troubles de réserve des glycosaminoglycanes provoqués par une déficience en iduronidase.

5. Enzyme α-L-iduronidase humaine recombinante suivant l'une quelconque des revendications 1 à 4, ayant une activité spécifique supérieure à environ 240 000 unités par milligramme de protéine.

6. Composition pharmaceutique comprenant l'enzyme α-L-iduronidase humaine recombinante de l'une quelconque des revendications 1 à 5 en association avec un support pharmaceutiquement acceptable.

7. Composition pharmaceutique suivant la revendication 6, comprenant en outre une solution de chlorure de sodium, un tampon et du polysorbate 80.

8. Composition pharmaceutique suivant la revendication 6 ou 7, dans laquelle ladite enzyme α-L-iduronidase humaine recombinante est présente à une concentration d'environ 0,5 mg/ml et d'environ 125 000 unités par ml.

9. Composition pharmaceutique suivant la revendication 7 ou 8, dans laquelle ladite solution de chlorure de sodium est présente à une concentration d'environ 150 mM.

10. Composition pharmaceutique suivant l'une quelconque des revendications 7 à 9, dans laquelle ledit tampon est un tampon au phosphate de sodium à une concentration d'environ 100 mM, et un pH de 5,4 à 5,9.

11. Composition pharmaceutique suivant l'une quelconque des revendications 7 à 10, dans laquelle, après dilution dans la forme posologique, l'albumine humaine est présente à une concentration d'au moins environ 1 mg/ml.

12. Composition pharmaceutique suivant l'une quelconque des revendications 7 à 11, dans laquelle le pH de ladite solution est maintenu à environ 5,8.

13. Composition pharmaceutique suivant l'une quelconque des revendications 7 à 12, dans laquelle ledit polysorbate 80 est maintenu à 0,001 %.


### SEQUENCE LISTING

<110> BIOMARIN PHARMACEUTICAL INC HARBOR-UCLA RESEARCH AND EDUCATION INSITTUTE HENSTRAND, JOHN
<120> RECOMBINANT ALPHA-L-IDURONIDASE, METHODS FOR PRODUCING AND PURIFYING THE SAME AND METHODS FOR TREATING DISEASES CADSED BY DEFICIENCIES THEREOF
<130> 00800005100PC00
<140> TO EE ASSIGNED
   <141> TO BE ASSIGNED
<150> 09/439,923
   <151> 1999-11-12
<160> 2
<170> FastSEQ for Windows Version 3.0
<210> 1
   <211> 6200
   <212> DNA
   <213> Homo sapiens
<220>
   <221> CDS
   <222> (1558)...(3510)
<400> 1
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttaact ggcttatcga aattaatacg actcactata gggagaccca agcttcgcag 900
   aattcctgcg gctgctacag tgtgtccagc gtcctgcctg gctgtgctga gcgctggaac 960
   agtggcgcat cattcaagtg cacagttacc catcctgagt ctggcacctt aactggcaca 1020
   attgccaaag tcacaggtga gctcagatgc ataccaggac attgtatgac gttccctgct 1080
   cacatgcctg ctttcttcct ataatacaga tgctcaacta actgctcatg tccttatatc 1140
   acagagggaa attggagcta tctgaggaac tgcccagaag ggaagggcag aggggtcttg 1200
   ctctccctgt ctgagccata actcttcttt ctaccttcca gtgaacacct tcccacccca 1260
   ggtccacctg ctaccgccgc cgtcggagga gctggccctg aatgagctct tgtccctgac 1320
   atgcctggtg cgagctttca accctaaaga agtgctggtg cgatggctgc atggaaatga 1380
   ggagctgtcc ccagaaagct acctagtgtt tgagccccta aaggagccag gcgagggagc 1440
   caccacctac ctggtgacaa gcgtgttgcg tgtatcagct gaaagcttga tatcgaattc   1500
   cggaggcgga accggcagtg cagcccgaag ccccgcagtc cccgagcacg cgtggcc atg   1560
                                                                  Met
                                                                   1
   cgt ccc ctg cgc ccc cgc gcc gcg ctg ctg gcg ctc ctg gcc tcg ctc      1608
   Arg Pro Leu Arg Pro Arg Ala Ala Leu Leu Ala Leu Leu Ala Ser Leu
               5                   10                  15
   ctg gcc gcg ccc ccg gtg gcc ccg gcc gag gcc ccg cac ctg gtg cat      1656
   Leu Ala Ala Pro Pro Val Ala Pro Ala Glu Ala Pro His Leu Val His
           20                  25                  30
   gtg gac gcg gcc cgc gcg ctg tgg ccc ctg cgg cgc ttc tgg agg agc      1704
   Val Asp Ala Ala Arg Ala Leu Trp Pro Leu Arg Arg Phe Trp Arg Ser
       35                  40                  45
   aca ggc ttc tgc ccc ccg ctg cca cac agc cag gct gac cag tac gtg      1752
   Thr Gly Phe Cys Pro Pro Leu Pro His Ser Gln Ala Asp Gln Tyr Val
   50                  55                  60                  65
   ctc agc tgg gac cag cag ctc aac ctc gcc tat gtg ggc gcc gtc cct      1800
   Leu Ser Trp Asp Gln Gln Leu Asn Leu Ala Tyr Val Gly Ala Val Pro
                   70                  75                  80
   cac cgc ggc atc aag cag gtc cgg acc cac tgg ctg ctg gag ctt gtc      1848
   His Arg Gly Ile Lys Gln Val Arg Thr His Trp Leu Leu Glu Leu Val
               85                  90                  95
   acc acc agg ggg tcc act gga cgg ggc ctg agc tac aac ttc acc cac      1896
   Thr Thr Arg Gly Ser Thr Gly Arg Gly Leu Ser Tyr Asn Phe Thr His
           100                 105                 110
   ctg gac ggg tac ctg gac ctt ctc agg gag aac cag ctc ggg ttt gag      1944
   Leu Asp Gly Tyr Leu Asp Leu Leu Arg Glu Asn Gln Leu Gly Phe Glu
       115                 120                 125
   ctg atg ggc agc gcc tcg ggc cac ttc act gac ttt gag gac aag cag      1992
   Leu Met Gly Ser Ala Ser Gly His Phe Thr Asp Phe Glu Asp Lys Gln
   130                 135                 140                 145
   cag gtg ttt gag tgg aag gac ttg gtc tcc agc ctg gcc agg aga tac      2040
   Gln Val Phe Glu Trp Lys Asp Leu Val Ser Ser Leu Ala Arg Arg Tyr
                   150                 155                 160
   atc ggt agg tac gga ctg gcg cat gtt tcc aag tgg aac ttc gag acg      2088
   Ile Gly Arg Tyr Gly Leu Ala His Val Ser Lys Trp Asn Phe Glu Thr
               165                 170                 175
   tgg aat gag cca gac cac cac gac ttt gac aac gtc tcc atg acc atg      2136
   Trp Asn Glu Pro Asp His His Asp Phe Asp Asn Val Ser Met Thr Met
           180                 185                 190
   caa ggc ttc ctg aac tac tac gat gcc tgc tcg gag ggt ctg cgc gcc      2184
   Gln Gly Phe Leu Asn Tyr Tyr Asp Ala Cys Ser Glu Gly Leu Arg Ala
       195                 200                 205
   gcc agc ccc gcc ctg cgg ctg gga ggc ccc ggc gac tcc ttc cac acc     2232
   Ala Ser Pro Ala Leu Arg Leu Gly Gly Pro Gly Asp Ser Phe His Thr
   210                 215                 220                 225
   cca ccg cga tcc ccg ctg agc tgg ggc ctc ctg cgc cac tgc cac gac     2280
   Pro Pro Arg Ser Pro Leu Ser Trp Gly Leu Leu Arg His Cys His Asp
                   230                 235                 240
   ggt acc aac ttc ttc act ggg gag gcg ggc gtg cgg ctg gac tac atc     2328
   Gly Thr Asn Phe Phe Thr Gly Glu Ala Gly Val Arg Leu Asp Tyr Ile
               245                 250                 255
   tcc ctc cac agg aag ggt gcg cgc agc tcc atc tcc atc ctg gag cag     2376
   Ser Leu His Arg Lys Gly Ala Arg Ser Ser Ile Ser Ile Leu Glu Gln
           260                 265                 270
   gag aag gtc gtc gcg cag cag atc cgg cag ctc ttc ccc aag ttc gcg     2424
   Glu Lys Val Val Ala Gln Gln Ile Arg Gln Leu Phe Pro Lys Phe Ala
       275                 280                 285
   gac acc ccc att tac aac gac gag gcg gac ccg ctg gtg ggc tgg tcc     2472
   Asp Thr Pro Ile Tyr Asn Asp Glu Ala Asp Pro Leu Val Gly Trp Ser
   290                 295                 300                 305
   ctg cca cag ccg tgg agg gcg gac gtg acc tac gcg gcc atg gtg gtg     2520
   Leu Pro Gln Pro Trp Arg Ala Asp Val Thr Tyr Ala Ala Met Val Val
                   310                 315                 320
   aag gtc atc gcg cag cat cag aac ctg cta ctg gcc aac acc acc tcc     2568
   Lys Val Ile Ala Gln His Gln Asn Leu Leu Leu Ala Asn Thr Thr Ser
               325                 330                 335
   gcc ttc ccc tac gcg ctc ctg agc aac gac aat gcc ttc ctg agc tac     2616
   Ala Phe Pro Tyr Ala Leu Leu Ser Asn Asp Asn Ala Phe Leu Ser Tyr
           340                 345                 350
   cac ccg cac ccc ttc gcg cag cgc acg ctc acc gcg cgc ttc cag gtc     2664
   His Pro His Pro Phe Ala Gln Arg Thr Leu Thr Ala Arg Phe Gln Val
       355                 360                 365
   aac aac acc cgc ccg ccg cac gtg cag ctg ttg cgc aag ccg gtg ctc     2712
   Asn Asn Thr Arg Pro Pro His Val Gln Leu Leu Arg Lys Pro Val Leu
   370                 375                 380                 385
   acg gcc atg ggg ctg ctg gcg ctg ctg gat gag gag cag ctc tgg gcc     2760
   Thr Ala Met Gly Leu Leu Ala Leu Leu Asp Glu Glu Gln Leu Trp Ala
                   390                 395                 400
   gaa gtg tcg cag gcc ggg acc gtc ctg gac agc aac cac acg gtg ggc     2808
   Glu Val Ser Gln Ala Gly Thr Val Leu Asp Ser Asn His Thr Val Gly
               405                 410                 415
   gtc ctg gcc agc gcc cac cgc ccc cag ggc ccg gcc gac gcc tgg cgc     2856
   Val Leu Ala Ser Ala His Arg Pro Gln Gly Pro Ala Asp Ala Trp Arg
           420                 425                 430
   gcc gcg gtg ctg atc tac gcg agc gac gac acc cgc gcc cac ccc aac     2904
   Ala Ala Val Leu Ile Tyr Ala Ser Asp Asp Thr Arg Ala His Pro Asn
   435 440 445
   cgc agc gtc gcg gtg acc ctg cgg ctg cgc ggg gtg ccc ccc ggc ccg 2952
   Arg Ser Val Ala Val Thr Leu Arg Leu Arg Gly Val Pro Pro Gly Pro
   450 455 460 465
   ggc ctg gtc tac gtc acg cgc tac ctg gac aac ggg ctc tgc agc ccc 3000
   Gly Leu Val Tyr Val Thr Arg Tyr Leu Asp Asn Gly Leu Cys Ser Pro
   470 475 480
   gac ggc gag tgg cgg cgc ctg ggc cgg ccc gtc ttc ccc acg gca gag 3048
   Asp Gly Glu Trp Arg Arg Leu Gly Arg Pro Val Phe Pro Thr Ala Glu
   485 490 495
   cag ttc cgg cgc tag cgc gcg gct gag gac ccg gtg gcc gcg gcg ccc 3096
   Gln Phe Arg Arg * Arg Ala Ala Glu Asp Pro Val Ala Ala Ala Pro
   500 505 510
   cgc ccc tta ccc gcc ggc ggc cgc ctg agg ctg cgc ccc gcg ctg cgg 3144
   Arg Pro Leu Pro Ala Gly Gly Arg Leu Arg Leu Arg Pro Ala Leu Arg
   515 520 525
   ctg ccg tcg ctt ttg ctg gtg cac gtg tgt gcg cgc ccc gag aag ccg 3192
   Leu Pro Ser Leu Leu Leu Val His Val Cys Ala Arg Pro Glu Lys Pro
   530 535 540
   ccc ggg cag gtc acg cgg ctc cgc gcc ctg ccc ctg acc caa ggg cag 3240
   Pro Gly Gln Val Thr Arg Leu Arg Ala Leu Pro Leu Thr Gln Gly Gln
   545 550 555 560
   ctg gtt ctg gtc tgg tcg gat gaa cac gtg ggc tcc aag tgc ctg tgg 3288
   Leu Val Leu Val Trp Ser Asp Glu His Val Gly Ser Lys Cys Leu Trp
   565 570 575
   aca tac gag atc cag ttc tct cag gac ggt aag gcg tac acc ccg gtc 3336
   Thr Tyr Glu Ile Gln Phe Ser Gln Asp Gly Lys Ala Tyr Thr Pro Val
   580 585 590
   agc agg aag cca tcg acc ttc aac ctc ttt gtg ttc agc cca gac aca 3384
   Ser Arg Lys Pro Ser Thr Phe Asn Leu Phe Val Phe Ser Pro Asp Thr
   595 600 605
   ggt gct gtc tct ggc tcc tac cga gtt cga gcc ctg gac tac tgg gcc 3432
   Gly Ala Val Ser Gly Ser Tyr Arg Val Arg Ala Leu Asp Tyr Trp Ala
   610 615 620
   cga cca ggc ccc ttc tcg gac cct gtg ccg tac ctg gag gtc cct gtg 3480
   Arg Pro Gly Pro Phe Ser Asp Pro Val Pro Tyr Leu Glu Val Pro Val
   625 630 635 640
   cca aga ggg ccc cca tcc ccg ggc aat cca tgagcctgtg ctgagcccca 3530
   Pro Arg Gly Pro Pro Ser Pro Gly Asn Pro
   645 650
   gtgggttgca cctccaccgg cagtcagcga gctggggctg cactgtgccc atgctgccct 3590
   cccatcaccc cctttgcaat atatttttat attttaaaaa aaaaaaaaaa aaaaaaaaaa 3650
   aaaaaaaaaa aaaaaaaaaa aaaaaaaaaa aaaaaaaaaa aaaaaaaaag aattcctgca    3710
   gccttctagt tgccagccat ctgttgtttg cccctccccc gtgccttcct tgaccctgga    3770
   aggtgccact cccactgtcc tttcctaata aaatgaggaa attgcatcgc attgtctgag    3830
   taggtgtcat tctattctgg ggggtggggt ggggcaggac agcaaggggg aggattggga    3890
   agacaatagc aggcatgctg gggatgcggt gggctctatg gcttctgagg cggaaagaac    3950
   cagctggggc tcgagagctt ggcgtaatca tggtcatagc tgtttcctgt gtgaaattgt    4010
   tatccgctca caattccaca caacatacga gccggaagca taaagtgtaa agcctggggt    4070
   gcctaatgag tgagctaact cacattaatt gcgttgcgct cactgcccgc tttccagtcg    4130
   ggaaacctgt cgtgccagct gcattaatga atcggccaac gcgcggggag aggcggtttg    4190
   cgtattgggc gctcttccgc ttcctcgctc actgactcgc tgcgctcggt cgttcggctg    4250
   cggcgagcgg tatcagctca ctcaaaggcg gtaatacggt tatccacaga atcaggggat    4310
   aacgcaggaa agaacatgtg agcaaaaggc cagcaaaagg ccaggaaccg taaaaaggcc    4370
   gcgttgctgg cgtttttcca taggctccgc ccccctgacg agcatcacaa aaatcgacgc    4430
   tcaagtcaga ggtggcgaaa cccgacagga ctataaagat accaggcgtt tccccctgga    4490
   agctccctcg tgcgctctcc tgttccgacc ctgccgctta ccggatacct gtccgccttt    4550
   ctcccttcgg gaagcgtggc gctttctcaa tgctcacgct gtaggtatct cagttcggtg    4610
   taggtcgttc gctccaagct gggctgtgtg cacgaacccc ccgttcagcc cgaccgctgc    4670
   gccttatccg gtaactatcg tcttgagtcc aacccggtaa gacacgactt atcgccactg    4730
   gcagcagcca ctggtaacag gattagcaga gcgaggtatg taggcggtgc tacagagttc    4790
   ttgaagtggt ggcctaacta cggctacact agaaggacag tatttggtat ctgcgctctg    4850
   ctgaagccag ttaccttcgg aaaaagagtt ggtagctctt gatccggcaa acaaaccacc    4910
   gctggtagcg gtggtttttt tgtttgcaag cagcagatta cgcgcagaaa aaaaggatct    4970
   caagaagatc ctttgatctt ttctacgggg tctgacgctc agtggaacga aaactcacgt    5030
   taagggattt tggtcatgag attatcaaaa aggatcttca cctagatcct tttaaattaa    5090
   aaatgaagtt ttaaatcaat ctaaagtata tatgagtaaa cttggtctga cagttaccaa    5150
   tgcttaatca gtgaggcacc tatctcagcg atctgtctat ttcgttcatc catagttgcc    5210
   tgactccccg tcgtgtagat aactacgata cgggagggct taccatctgg ccccagtgct    5270
   gcaatgatac cgcgagaccc acgctcaccg gctccagatt tatcagcaat aaaccagcca    5330
   gccggaaggg ccgagcgcag aagtggtcct gcaactttat ccgcctccat ccagtctatt    5390
   aattgttgcc gggaagctag agtaagtagt tcgccagtta atagtttgcg caacgttgtt    5450
   gccattgcta caggcatcgt ggtgtcacgc tcgtcgtttg gtatggcttc attcagctcc    5510
   ggttcccaac gatcaaggcg agttacatga tcccccatgt tgtgcaaaaa agcggttagc    5570
   tccttcggtc ctccgatcgt tgtcagaagt aagttggccg cagtgttatc actcatggtt    5630
   atggcagcac tgcataattc tcttactgtc atgccatccg taagatgctt ttctgtgact    5690
   ggtgagtact caaccaagtc attctgagaa tagtgtatgc ggcgaccgag ttgctcttgc    5750
   ccggcgtcaa tacgggataa taccgcgcca catagcagaa ctttaaaagt gctcatcatt    5810
   ggaaaacgtt cttcggggcg aaaactctca aggatcttac cgctgttgag atccagttcg    5870
   atgtaaccca ctcgtgcacc caactgatct tcagcatctt ttactttcac cagcgtttct    5930
   gggtgagcaa aaacaggaag gcaaaatgcc gcaaaaaagg gaataagggc gacacggaaa    5990
   tgttgaatac tcatactctt cctttttcaa tattattgaa gcatttatca gggttattgt    6050
   ctcatgagcg gatacatatt tgaatgtatt tagaaaaata aacaaatagg ggttccgcgc    6110
   ctcatgagcg gatacatatt tgaatgtatt tagaaaaata aacaaatagg ggttccgcgc    6170
   acatttcccc gaaaagtgcc acctgacgtc                                      6200
<210> 2
   <211> 650
   <212> PRT
   <213> Homo sapiens
<400> 2